(19)

Europäisches
Patentamt

European
Patent Office

Office européen
des brevets

(11)     **EP 4 260 907 A1**

(12)                    **EUROPEAN PATENT APPLICATION**

(43) Date of publication:
    **18.10.2023  Bulletin 2023/42**

(21) Application number: **22167625.7**

(22) Date of filing: **11.04.2022**

(51) International Patent Classification (IPC):
    ***A61P 15/00*** (2006.01)      ***C07K 16/28*** (2006.01)
    ***C07K 16/40*** (2006.01)

(52) Cooperative Patent Classification (CPC):
    **A61P 15/00; C07K 16/2803; C07K 16/283;
    C07K 16/40;** C07K 2317/22; C07K 2317/569;
    C07K 2317/622; C07K 2319/50

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**
    Designated Validation States:
    **KH MA MD TN**

(71) Applicant: **Universität Zürich
    8006 Zürich (CH)**

(72) Inventors:
    • **VONGRAD, Valentina
      8006 Zürich (CH)**
    • **SHILAIH, Mohaned
      8006 Zürich (CH)**
    • **LEENERS, Brigitte
      8006 Zürich (CH)**

(74) Representative: **Vossius & Partner
    Patentanwälte Rechtsanwälte mbB
    Siebertstraße 3
    81675 München (DE)**

Remarks:
    The complete document including Reference
    Table(s) and the Sequence Listing(s) can be
    downloaded from the EPO website

(54)   **AGENTS FOR TREATMENT OF ENDOMETRIOSIS AND OTHER BENIGN GYNECOLOGICAL
       NEOPLASMS**

(57)     The present invention relates to agents for the treatment and/or diagnosis of endometriosis and other benign gynecological neoplasms disease or disorder and also relates to agents capable of eliciting a cytotoxic response in benign gynecological neoplasms diseases or disorders.

EP 4 260 907 A1

**Description**

The present invention relates to agents for the treatment and/or diagnosis of endometriosis and other benign gynecological neoplasms disease or disorder and also relates to agents capable of eliciting a cytotoxic response in benign gynecological neoplasms diseases or disorders.

Background of the Invention

Benign gynecological neoplasms share an overrepresentation of fibroblasts, examples of such diseases include endometriosis and uterine fibroids (myoma). Endometriosis has been classically defined as the ectopic presence of endometrial epithelial and stromal cells. However, there is mounting evidence from recent literature that such a definition falls short, due to the low representation of epithelial cells in some lesions. The same studies point towards the fibrotic nature of the disease as a consistent feature of all stages of the disease. This has led to several calls to redefine endometriosis as a pro-fibrotic disease (P Vigano, et al., Human Reproduction, Volume 33, Issue 3, March 2018, Pages 347-352; Sun-Wei Guo, Patrick G Groothuis, Human Reproduction Update, Volume 24, Issue 5, September-October 2018, Pages 577-598). Attempts to tackle the fibrotic and inflammatory pathways of endometriosis through TNF-a and VEGF targeting or other pathway interferences had limited success pre-clinically and in the clinic. Given that repeated tissue injury and repair leads to irreversible differentiation of fibroblasts to myofibroblasts, such terminal differentiation limits the fibroblasts' reactivity to temporary changes in their signaling pathways (e.g., anti- TNF-a or TGF-B). A similar picture is present in myoma.
FAP is part of a larger family of prolyl-specific serine proteases which has both enzymatic and signaling roles in health and disease. The protein has been associated with fibrosis, cancers, and inflammation in various organs, however, it is rarely expressed in normal tissue rendering it an attractive target for treatments. This is because FAP is only expressed in tissues undergoing active injury and repair. FAP, is also known to be affected by estrogen expression in cancer, and estrogen is one of main drivers of endometriosis. In addition, FAP has been shown to be involved in the PTEN/PI3K/AKT and Ras-ERK pathways which are some of the most common mutation-bearing genes observed in Endometriosis.
Based on the above-mentioned state of the art, the objective of the present invention is to provide means and methods for treatment of benign gynecological neoplasm disease or disorder such as endometriosis and myoma. This objective is attained by the subject-matter of the independent claims of the present specification, with further advantageous embodiments described in the dependent claims, examples, figures and general description of this specification.

Summary of the Invention

The inventors describe for the first time the overexpression of FAP and its use in treatment of endometriosis and associated adhesions and other benign gynecological neoplasms (e.g., myoma).
A first aspect of the invention relates to an agent, the agent comprising

    a. a ligand/targeting moiety capable of specifically binding to FAP; and
    b. a therapeutic moiety, wherein the therapeutic moiety is capable of triggering cell death of cells expressing FAP on their cell surface directly, or indirectly via recruiting immune cells;
    c. wherein optionally, the ligand and the immune cell recruiting moiety are attached (covalently linked) to each other via a peptidic linker,

particularly for use in treatment of a benign gynecological neoplastic disease or disorder.
A second aspect of the invention relates to an agent, wherein the agent comprises

    a. a target protein binding moiety (wherein the target protein is overexpressed in benign gynecological neoplasm cells);
    b. a biocidal molecule, a cytotoxic molecule, a radioisotope and/or an immune cell recruiting moiety, wherein the immune-cell recruiting moiety is capable of specifically binding to an immune cell surface molecule (wherein the immune cell recruiting moiety is capable of recruiting immune cells, particularly CD8[+] T-cells or NK cells);
    c. a masking moiety capable of shielding the immune cell recruiting moiety and/or the target protein binding moiety from binding to its target;
    d. a cleavable linker connecting the masking moiety with the other components of the agent, wherein the cleavable linker is specifically cleavable (recognizable) by matrix metalloproteinases (MMPs) 2, 9, 10, and/or 26;

particularly for use in treatment of a benign gynecological neoplasm.

A third aspect of the invention relates to an agent as specified in any one of the preceding aspects.

A fourth aspect of the invention relates to an agent comprising

a. a ligand capable of specifically binding to FAP;

b. a radioisotope label,

particularly for use in diagnosis of a benign gynecological neoplasm.

*Terms and definitions*

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *FAP* in the context of the present specification relates to Prolyl endopeptidase FAP or Fibroblast activation protein alpha (UniProt ID Q12884)

The term CD3 in the context of the present specification relates to T-cell surface glycoprotein CD3 epsilon chain individually (UniProt ID P07766) and in its naturally occurring in human heterodimer forms (CD3 epsilon and CD3 delta (UniProt ID P04234) heterodimer, CD3 epsilon and CD3 gamma heterodimer (UniProt ID P09693), and CD3 epsilon and CD3 zeta hetero (UniProt ID P20963) dimer)

The term CD16a in the context of the present specification relates to Low affinity immunoglobulin gamma Fc region receptor III-A (UniProt ID P08637).

The term CD8 in the context of the present specification relates to T-cell surface glycoprotein CD8 alpha (UniProt ID P01732), T-cell surface glycoprotein CD8 beta (UniProt ID P10966), and their naturally occurring in human heterodimer (CD8 beta and CD8 alpha heterodimer)

The term *Okt3* in the context of the present specification relates to a recombinant antibody targeting CD3 as described in US6750325B1, which is incorporated by reference herein.

*Sequences*

Sequences similar or homologous (e.g., at least about 70% sequence identity) to the sequences disclosed herein are also part of the invention. In some embodiments, the sequence identity at the amino acid level can be about 80%, 85%, 90%, 91%, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

Reference to identical sequences without specification of a percentage value implies 100% identical sequences (i.e. the same sequence).

*General Biochemistry: Peptides, Amino Acid Sequences*

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

The term *peptide* in the context of the present specification relates to a molecule consisting of up to 50 amino acids, in particular 8 to 30 amino acids, more particularly 8 to 15amino acids, that form a linear chain wherein the amino acids are connected by peptide bonds.

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

In the context of the present specification, the term *amino acid linker or peptidic linker* refers to a polypeptide of variable length that is used to connect two polypeptides in order to generate a single chain polypeptide. Exemplary embodiments of linkers useful for practicing the invention specified herein are oligopeptide chains consisting of 1, 2, 3, 4, 5, 10, 20, 30, 40 or 50 amino acids. A non-limiting example of an amino acid linker is a monomer or di-, tri-or tetramer of a tetraglycine-serine peptide linker.

*General Molecular Biology: Nucleic Acid Sequences, Expression*

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of a RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

*Binding; Binders Ligands Antibodies:*

The term *specific binding* in the context of the present invention refers to a property of ligands that bind to their target with a certain affinity and target specificity. The affinity of such a ligand is indicated by the dissociation constant of the ligand. A specifically reactive ligand has a dissociation constant of $\leq 5*10^{-7}$ mol/L, particularly $\leq 10^{-8}$ mol/L, more particularly $\leq 10^{-9}$ mol/L, when binding to its target, but a dissociation constant at least three orders of magnitude higher in its interaction with a molecule having a globally similar chemical composition as the target, but a different three-dimensional structure.

The term *aptamer* relates to an oligonucleotide or peptide molecule that binds to a specific target molecule. Aptamers can be created by selecting them from a large random sequence pool. Nucleic acid aptamers can be generated through repeated rounds of in-vitro selection or equivalently, SELEX (systematic evolution of ligands by exponential enrichment) to bind to molecular targets such as small molecules, proteins or nucleic acids through non-covalent interactions. Aptamers offer molecular recognition properties that rival that of antibodies.

In the context of the present specification, the term *antibody* refers to whole antibodies including but not limited to immunoglobulin type G (IgG), type A (IgA), type D (IgD), type E (IgE) or type M (IgM), any antigen-binding fragment or single chains thereof and related or derived constructs. A whole antibody is a glycoprotein comprising at least two heavy (H) chains and two light (L) chains inter-connected by disulfide bonds. Each heavy chain is comprised of a heavy chain variable region ($V_H$) and a heavy chain constant region ($C_H$). The heavy chain constant region of IgG is comprised of three domains, $C_H1$, $C_H2$ and $C_H3$. Each light chain is comprised of a light chain variable region (abbreviated herein as $V_L$) and a light chain constant region ($C_L$). The light chain constant region is comprised of one domain, $C_L$. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component of the classical complement system. Similarly, the term encompasses a so-called nanobody or single domain antibody, an antibody fragment consisting of a single monomeric variable antibody domain.

In the context of the present specification, the term *humanized antibody* refers to an antibody originally produced by immune cells of a non-human species, the protein sequences of which have been modified to increase their similarity to antibody variants produced naturally in humans. The term *humanized antibody* as used herein includes antibodies in which CDR sequences derived from the germline of another mammalian species, such as a llama, have been grafted onto human framework sequences. Additional framework region modifications may be made within the human framework sequences as well as within the CDR sequences derived from the germline of another mammalian species.

The term *antibody-like molecule* in the context of the present specification refers to a molecule capable of specific binding to another molecule or target with high affinity / a Kd $\leq$ 10E-6 mol/l. An antibody-like molecule binds to its target similarly to the specific binding of an antibody. The term *antibody-like molecule* encompasses a repeat protein, such as a designed ankyrin repeat protein (Molecular Partners, Zürich), an engineered antibody mimetic protein exhibiting highly specific and high-affinity target protein binding (see US2012142611, US2016250341, US2016075767 and US2015368302, all of which are incorporated herein by reference). The term antibody-like molecule further encompasses, but is not limited to, a polypeptide derived from armadillo repeat proteins, a polypeptide derived from leucine-rich repeat proteins and a polypeptide derived from tetratricopeptide repeat proteins. The term *antibody-like molecule* further encompasses a specifically binding polypeptide derived from a protein A domain, a fibronectin domain FN3, a consensus fibronectin domain, a lipocalin (see Skerra, Biochim. Biophys. Acta 2000, 1482(1-2):337-50), a polypeptide derived from a Zinc finger protein (see Kwan et al. Structure 2003, 11(7):803-813), a Src homology domain 2 (SH2) or Src homology domain 3 (SH3), a PDZ domain, a gamma-crystallin, ubiquitin, a cysteine knot polypeptide or a knottin, cystatin, Sac7d, a triple helix coiled coil (also known as alphabodies), a Kunitz domain or a Kunitz-type protease inhibitor and a carbohydrate binding module 32-2.

The term *protein A domains derived polypeptide* refers to a molecule that is a derivative of protein A and is capable of specifically binding the Fc region and the Fab region of immunoglobulins.

The term *armadillo repeat protein* refers to a polypeptide comprising at least one armadillo repeat, wherein an armadillo repeat is characterized by a pair of alpha helices that form a hairpin structure.

The term *single domain antibody* refers to a heavy chain only antibody, VHH, or nanobody.

The term *single-chain variable fragment (scFv)* refers to recombinant molecules in which the variable regions of light and heavy immunoglobulin chains encoding antigen-binding domains are engineered into a single polypeptide.

The term *humanized camelid antibody* in the context of the present specification refers to an antibody consisting of only the heavy chain or the variable domain of the heavy chain (VHH domain) and whose amino acid sequence has been modified to increase their similarity to antibodies naturally produced in humans and, thus show a reduced immunogenicity when administered to a human being. A general strategy to humanize camelid antibodies is shown in Vincke et al. "General strategy to humanize a camelid single-domain antibody and identification of a universal humanized nanobody scaffold", J Biol Chem. 2009 Jan 30;284(5):3273-3284, and US2011165621A1.

In the context of the present specification, the term *fragment crystallizable (Fc) region* is used in its meaning known in the art of cell biology and immunology; it refers to a fraction of an antibody comprising, if applied to IgG, two identical heavy chain fragments comprised of a $C_H2$ and a $C_H3$ domain, covalently linked by disulfide bonds.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. endometriosis) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

The term *immune cells* in the context of the present specification relates to cells of the immune system which are capable of (directly or indirectly) killing cells of the body and/or inducing their apoptosis. Examples of immune cells are CD8+ cells or natural killer cells (NK cells).

The term *immune cell surface molecule* in the context of the present specification relates to a molecule, particularly a protein, which is located at the cell surface of an immune cell and which is accessible to other moieties.

The term *recruiting immune cells* in the context of the present specification relates to the ability of the molecule of the invention to bring immune cells in close contact with endometriotic cells, thereby recruiting the immune cells.

The term *bulk protein* in the context of the present specification relates to any protein present in bulk quantity in the blood (>1% of blood). Examples for a bulk protein are albumin, globulins, fibronogens.

The term *detectable expression* in the context of the present specification relates to the amount of protein expressed by a cell. If the protein is detectable on the cell surface of at least a subset by standard cell biology methods, it is considered as detectable expression.

The term *shielding* in the context of the present specification relates to a property of the masking moiety. The masking moiety is able to non-covalently interact with a certain moiety of the agent, and thereby prevent this other moiety from binding to its actual target as long as the masking moiety is covalently attached to the agent.

The term *endometriosis or ectopic endometrium* in the context of the present specification relates to any disease in which cells similar to those in the endometrium, the layer of tissue that normally covers the inside of the uterus, grow outside the uterus. The most common sites of endometriosis are the ovaries, followed by the Douglas pouch, the posterior leaves of the broad ligaments, and the sacro-uterine ligaments. Endometriosis in this invention relates to all subtypes or alternative naming including but not limited to adenomyosis, endometrioma, deep infiltrating endometriosis, superficial peritoneal endometriosis, and abdominal wall endometriosis.

The term *uterine fibroids, leiomyoma,* or *myoma* are fibrotic and uterine muscle outgrowths in and around the female reproductive organ. Uterine fibroids often co-occur with endometriosis. The major cell population of Uterine fibroids are similar to endometriosis but the initiating cells are uterine muscle cells, in contrast to endometrial epithelial cells in endometriosis.

The term *benign gynecological neoplasm or neoplastic disease* refers to abnormal growth of cells of noncancerous nature. Where benign gynecological neoplasm describes a case where tissue normally found in and around the female reproductive organ, grow abnormally on the organ itself or other organs. Endometriosis as an example is an abnormal growth of endometrial like cellular population on other organs that then cause pain and other symptoms for the patient. Myoma or uterine fibroids are other examples of benign gynecological neoplasms.

Detailed Description of the Invention

A first aspect of the invention relates to an agent, the agent comprising

  a. a ligand/targeting moiety capable of specifically binding to FAP; and
  b. a therapeutic moiety, wherein the therapeutic moiety is capable of triggering cell death of cells expressing FAP on their cell surface directly, or indirectly via recruiting immune cells;
  c. wherein optionally, the ligand and the immune cell recruiting moiety are attached (covalently linked) to each other via a peptidic linker,

particularly for use in treatment of a benign gynecological neoplastic disease or disorder.

In certain embodiments, the therapeutic moiety is a biocidal molecule, a cytotoxic molecule, and/or an immune cell recruiting moiety, wherein the immune-cell recruiting moiety is capable of specifically binding to an immune cell surface molecule (wherein the immune cell recruiting moiety is capable of recruiting immune cells, particularly CD8$^+$ T-cells or NK cells).

In certain embodiments, the therapeutic moiety is an immune cell recruiting moiety, wherein the immune-cell recruiting moiety is capable of specifically binding to an immune cell surface molecule.

In certain embodiments, the immune cell surface molecule is selected from

    a) CD3;
    b) CD16;
    c) CD8;

In certain embodiments, the immune cell surface molecule is CD3.

In certain embodiments, the immune cell recruiting moiety is selected from the group comprising an antibody, an antibody fragment, a single-chain antigen-binding fragment, an aptamer, a non-immunoglobin scaffold, a small molecule, and an antibody-like molecule. In certain embodiments, the immune cell recruiting moiety is a single-domain antibody. In certain embodiments, the immune cell recruiting moiety is selected from the group comprising an antibody, an antibody fragment, a single-chain antigen-binding fragment. In certain embodiments, the immune-cell recruiting moiety is a single-domain antibody.

In certain embodiments, the therapeutic moiety is a cytotoxic molecule of molecular weight of <1000 Daltons (Da). In certain embodiments, the therapeutic moiety is a cytotoxic molecule of molecular weight ≤800Da. In certain embodiments, the therapeutic moiety is a cytotoxic molecule of molecular weight ≤500Da.

In certain embodiments, the cytotoxic molecule is selected from the group comprising doxorubicin, carminomycin, daunorubicin, aminopterin, methotrexate, methopterin, dichloro-methotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, leurosine, taxol, desacetylvinblastine, cyclophosphamide, ifosfamide, cytarabine, 6-thioguanine, chlorambucil, carmustine, mitoxantrone, paclitaxel or a cytotoxic derivative thereof.

In certain embodiments, the ligand capable of specifically binding to FAP is selected from the group comprising an antibody, an antibody fragment, a single-chain antigen-binding fragment, an aptamer, a non-immunoglobin scaffold, an antibody-like molecule, single-domain antibody, a small (≤1000Da, particularly ≤800Da, more particularly ≤500Da) molecule pharmaceutical obeying the Lipinski Rules of Five set of criteria, a small peptide, and a FAP specific substrate (e.g., (poly)peptide-Proline-Leu/Asn/aminotrifluoromethylcoumarin-(poly) peptide). In certain embodiments, the ligand is selected from the group comprising an antibody, an antibody fragment, a single-chain antigen-binding fragment. In certain embodiments, the ligand is a single-domain antibody.

In certain embodiments, the agent comprises a masking moiety which is attached (covalently linked) via a linker to the rest of the agent, wherein the masking moiety is capable of reducing or eliminating the binding ability of the ligand and/or the immune cell recruiting moiety (and preventing the immune-cell binding moiety or the ligand to bind to its target). In certain embodiments, the linker comprises a protease cleavage site (cleavable linker).

The masking approach is an addition that may allow for better therapeutic window.

In certain embodiments, the agent comprises a bulk protein binding site, wherein the binding site is attached (covalently linked) via a linker to the rest of the agent. In certain embodiments, the linker comprises a protease cleavage site. In certain embodiments, the bulk protein binding site is a human serum albumin binding site.

In certain embodiments, the protease cleavage site is specifically cleavable (recognizable) by matrix metalloproteinase 2, 9, 10, and/or 26.

In certain embodiments, two, three, or all components of the agent are covalently attached to each other via a peptide linker. In certain embodiments, each peptide linker sequence is independently selected from the sequences SEQ ID NO 5 to SEQ ID NO 15, ERK, $(GS)_n$, $(G_2S)_n$, $(G_3S)_n$, $(G_4S)_n$ with n being an integer selected from 1 to 10, wherein G is glycine, S is serine, E is glutamate, R is arginine, and K is lysine. In particular embodiments, n is 1, 2, 3, or 4.

In certain embodiments, the benign neoplasm tissue is characterized by detectable expression of the target protein. In certain embodiments, at least 1% of benign neoplasm cells express the target protein.

In certain embodiments, the ligand capable of specifically binding to FAP is characterized by a sequence of SEQ ID NO 16.

In certain embodiments, the immune cell surface molecule is CD3, and the therapeutic moiety is characterized by a sequence of SEQ ID NO 17.

A second aspect of the invention relates to an agent, wherein the agent comprises

    a. a target protein binding moiety (wherein the target protein is overexpressed in benign gynecological neoplasm cells);

b. a biocidal molecule, a cytotoxic molecule, a radioisotope and/or an immune cell recruiting moiety, wherein the immune-cell recruiting moiety is capable of specifically binding to an immune cell surface molecule (wherein the immune cell recruiting moiety is capable of recruiting immune cells, particularly CD8[+] T-cells or NK cells);

c. a masking moiety capable of shielding the immune cell recruiting moiety and/or the target protein binding moiety from binding to its target;

d. a cleavable linker connecting the masking moiety with the other components of the agent, wherein the cleavable linker is specifically cleavable (recognizable) by matrix metalloproteinases (MMPs) 2, 9, 10, and/or 26;

particularly for use in treatment of a benign gynecological neoplasm.

In certain embodiments of this second aspect of the invention, the cleavable linker is specifically cleavable (recognizable) by MMP 2 and/or 9.

In certain embodiments of the second aspect, the cleavable linker is characterized by a sequence selected from the group of SEQ ID NO 1-4.

In certain embodiments of the second aspect, the masking moiety is capable of shielding the immune cell recruiting moiety from binding its target.

In certain embodiments, the benign gynecological neoplastic disease is endometriosis. In certain embodiments, the benign gynecological neoplastic disease is occurrence of uterine fibroids.

In certain embodiments, the agent is formulated for subcutaneous or intravenous injection.

A third aspect of the invention relates to an agent as specified in any one of the preceding aspects.

A fourth aspect of the invention relates to an agent comprising

a. a ligand capable of specifically binding to FAP;

b. a radioisotope label,

particularly for use in diagnosis of a benign gynecological neoplasm.

In certain embodiments, the radioisotope label is selected from the group comprising $^{94m}$Tc, $^{186}$Re, $^{203}$Pb, $^{47}$Sc, $^{111}$In, $^{97}$Ru, $^{62}$Cu, $^{88}$Y, $^{121}$Sn, $^{161}$Tb, $^{153}$Sm, $^{166}$Ho, $^{105}$Rh.

In certain embodiments, the benign gynecological neoplastic disease is endometriosis. In certain embodiments, the benign gynecological neoplastic disease is occurrence of uterine fibroids.

The agent for as described above or a conjugate thereof may be for use in imaging, detecting, diagnosing benign neoplasms.

*Sequences*

| Linker cleavable by | Sequence | SEQ ID NO |
|---|---|---|
| MMP10 | PPAELVAD | 1 |
| MMP 2/9 | VHMPLGFLGP | 2 |
| MMP26.1 | KPLAYWAR | 3 |
| MMP26.2 | KPISLTAS | 4 |
| - | EPKSCDKTHT | 5 |
| - | CPPC | 6 |
| - | PAPELLGGP | 7 |
| - | CCVECPPC | 8 |
| - | PAPPVAGP | 9 |
| - | ELKTPLGDTTHT | 10 |
| - | CPRCP(EPKSCDTPPPCPRCP)3 | 11 |
| - | APELLGGP | 12 |

(continued)

| Linker cleavable by | Sequence | SEQ ID NO |
|---|---|---|
| - | ESKYGPP | 13 |
| - | CPSC | 14 |
| - | PAPEFLGGP | 15 |

| Construct scFV FAP | QVQLVQSGAEVKKPGASVKVSCKTSRYTFTEYTIHWVRQAPGQRLEWIGGINPNNGIPNYNQ KFKGRVTITVDTSASTAYMELSSLRSEDTAVYYCARRRIAYGYDEGHAMDYWGQGTLVTVSS *GGGGSGGGGSGGGGS* DIVMTQSPDSLAVSLGERATINCKSSQSLLYSRNQKN YLAWYQQKPGQPPKLLIFWASTRESGVPDRFSGSGFGTDFTLTISSLQAEDVAVYYCQQYFSYPLT FGQGTKVEIKDP (SEQ ID NO 16) **(heavy chain**-*linker*-light chain) |
|---|---|
| Construct scFV OKT3 | DIKLQQSGAELARPGASVKMSCKTSGYTFTRYTMHWVKQRPGQGLEWIGYINPSRGYTNYN QKFKDKATLTTDKSSSTAYMQLSSLTSEDSAVYYCARYYDDHYCLDYWGQGTTLTVSSVE *GGSGGGSGGSGGSGG* VDDIQLTQSPAIMSASPGEKVTMTCRASSSVSYMNWYQQ KSGTSPKRWIYDTSKVASGVPYRFSGSGSGTSYSLTISSMEAEDAATYYCQQWSSNPLTFGAGTK LELK (SEQ ID NO 17) **(heavy chain**-linker-light chain) |

*Medical treatment*

Similarly, within the scope of the present invention is a method for treating a benign gynecological neoplasm in a patient in need thereof, comprising administering to the patient an agent according to the above description.

In certain embodiments, the agent is an antibody, antibody fragment, an antibody-like molecule or a protein A domains derived polypeptide.

In some embodiments, the polypeptide agent is an immunoglobulin consisting of two heavy chains and two light chains. In some embodiments, the polypeptide ligand is a single domain antibody, consisting of an isolated variable domain from a heavy or light chain. In some embodiments, the non-agonist polypeptide ligand is a heavy-chain antibody consisting of only heavy chains such as antibodies found in camelids.

In certain embodiments, the agent is an antibody fragment. In certain embodiments, the polypeptide agent is a Fab fragment, i.e. the antigen-binding fragment of an antibody, or a single-chain variable fragment, i.e. a fusion protein of the variable region of heavy and the light chain of an antibody connected by a peptide linker.

*Pharmaceutical Compositions, Administration/Dosage Forms and Salts*

According to one aspect of the compound according to the invention, the compound according to the invention is provided as a pharmaceutical composition, pharmaceutical administration form, or pharmaceutical dosage form.

In certain embodiments of the invention, the compound of the present invention is typically formulated into pharmaceutical dosage forms to provide an easily controllable dosage of the drug and to give the patient an elegant and easily handleable product.

Similarly, a dosage form for the prevention or treatment of endometriosis is provided, comprising a non-agonist ligand or antisense molecule according to any of the above aspects or embodiments of the invention.

The invention further encompasses a pharmaceutical composition comprising a compound of the present invention, or a pharmaceutically acceptable salt thereof, and a pharmaceutically acceptable carrier. In further embodiments, the composition comprises at least two pharmaceutically acceptable carriers, such as those described herein.

Certain embodiments of the invention relate to a dosage form for parenteral administration, such as subcutaneous, intravenous, intrahepatic or intramuscular injection forms. Optionally, a pharmaceutically acceptable carrier and/or excipient may be present.

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

In certain embodiments, the pharmaceutical composition or combination of the present invention can be in unit dosage of about 1-1000 mg of active ingredient(s) for a subject of about 50-70 kg. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

*Method of Manufacture and Method of Treatment according to the invention*

The invention further encompasses, as an additional aspect, the use of the agent as identified herein, or its *pharmaceutically* acceptable salt, as specified in detail above, for use in a method of manufacture of a medicament for the treatment or prevention of benign gynecological neoplasms, particularly endometriosis or uterine fibroids.

Similarly, the invention encompasses methods of treatment of a patient having been diagnosed with a disease associated with benign gynecological neoplasms, particularly endometriosis or uterine fibroids. This method entails administering to the patient an effective amount of the agent as identified herein, or its *pharmaceutically* acceptable salt, as specified in detail herein.

Wherever alternatives for single separable features such as, for example, a linker type or ligand protein or medical indication are laid out herein as "embodiments", it is to be understood that such alternatives may be combined freely to form discrete embodiments of the invention disclosed herein. Thus, any of the alternative embodiments for a linker type may be combined with any of the alternative embodiments of ligand and these combinations may be combined with any medical indication mentioned herein.

The application further encompasses the following items:

*Items*

1. An agent comprising

    a. a ligand capable of specifically binding to FAP;
    and
    b. a therapeutic moiety, wherein the therapeutic moiety is capable of triggering cell death of cells expressing FAP on their cell surface;

    for use in treatment of a benign gynecological neoplastic disease.

2. The agent for use according to item 1, wherein the therapeutic moiety is an immune cell recruiting moiety, wherein the immune-cell recruiting moiety is capable of specifically binding to an immune cell surface molecule.

3. The agent for use according to item 2, wherein the immune cell surface molecule is selected from the group comprised of:

    a. CD3;
    b. CD16;
    c. CD8;

    particularly wherein the immune cell surface molecule is CD3.

4. The agent for use according to any one of the preceding items 2 or 3, wherein the immune-cell recruiting moiety is selected from the group comprising an antibody, an antibody fragment, a single-chain antigen-binding fragment, single-domain antibody, an aptamer, a non-immunoglobin scaffold, and an antibody-like molecule, particularly wherein the immune-cell recruiting moiety is selected from the group comprising an antibody, an antibody fragment, a single-chain antigen-binding fragment,

more particularly wherein the immune-cell recruiting moiety is a single-domain antibody.

5. The agent for use according to item 1, wherein the therapeutic moiety is a cytotoxic molecule of molecular weight of <1000 Daltons (Da), particularly of molecular weight ≤800Da, more particularly of molecular weight ≤500Da.

6. The agent for use according to item 5, wherein the cytotoxic molecule is selected from the group comprising doxorubicin, carminomycin, daunorubicin, aminopterin, methotrexate, methopterin, dichloro-methotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, leurosine, taxol, desacetylvinblastine, cyclophosphamide, ifosfamide, cytarabine, 6-thioguanine, chlorambucil, carmustine, mitoxantrone, paclitaxel or a cytotoxic derivative thereof.

7. The agent for use according to any one of the preceding items, wherein the ligand is selected from the group comprising an antibody, an antibody fragment, a single-chain antigen-binding fragment, single-domain antibody, an aptamer, a non-immunoglobin scaffold, and an antibody-like molecule, a small molecule pharmaceutical obeying the Lipinski Rules of Five set of criteria, a peptide, and a FAP specific substrate, particularly wherein the ligand is selected from the group comprising an antibody, an antibody fragment, a single-chain antigen-binding fragment, more particularly wherein the ligand is a single-domain antibody.

8. The agent for use according to any one of the preceding items, wherein the agent comprises a masking moiety which is attached via a linker to the agent, wherein the masking moiety is capable of reducing or eliminating the binding ability of the ligand and/or the immune cell recruiting moiety, particularly wherein the linker comprises a protease cleavage site.

9. The agent for use according to any one of the preceding items, wherein the agent comprises a bulk protein binding site, particularly a human serum albumin binding site, wherein the binding site is attached via a linker to the agent, particularly wherein the linker comprises a protease cleavage site.

10. The agent for use according to item 8 or 9, wherein the protease cleavage site is specifically cleavable by matrix metalloproteinase 2, 9, 10, and/or 26.

11. The agent for use according to any one of the preceding items, wherein two, three, or all components of the agent are covalently attached to each other via a peptide linker, particularly wherein each peptide linker sequence is independently selected from the sequences SEQ ID NO 5 to SEQ ID NO 15, ERK, $(GS)_n$, $(G_2S)_n$, $(G_3S)_n$, $(G_4S)_n$, with n being an integer selected from 1, 2, 3, 4, 5, 6, 7, 8, 9, and 10.

12. The agent for use according to any one of the preceding items, wherein the endometriotic tissue is characterized by detectable expression of FAP, particularly wherein at least 1% of endometriotic cells express FAP.

13. The agent for use according to any one of the preceding items, wherein the ligand capable of specifically binding to FAP is characterized by a sequence of SEQ ID NO 16.

14. The agent for use according to any one of the preceding items 2 to 4 or 7 to 13, wherein the immune cell surface molecule is CD3, and the therapeutic moiety is characterized by a sequence of SEQ ID NO 17.

15. An agent for use in treatment of a benign gynecological neoplastic disease, wherein the agent comprises

  a. a target protein binding moiety;
  b. an immune cell recruiting moiety;
  c. a masking moiety capable of shielding the immune cell recruiting moiety and/or the target protein binding moiety from binding to its target;
  d. a cleavable linker connecting the masking moiety with the other components of the agent, wherein the cleavable linker is specifically cleavable by matrix metalloproteinases (MMPs) 2, 9, 10, and/or 26.

16. The agent for use according to item 15, wherein the cleavable linker is specifically cleavable by MMP 2 or MMP 9.

17. The agent for use according to item 15 or 16, wherein the cleavable linker is characterized by a sequence selected from the group of SEQ ID NO 1 to SEQ ID NO 4.

18. The agent for use according to item 15 to 17, wherein the masking moiety is capable of shielding the immune cell recruiting moiety from binding its target.

19. The agent for use according to any one of the preceding items, wherein the benign gynecological neoplastic disease is endometriosis.

20. The agent for use according to any one of the preceding items 1 to 18, wherein the benign gynecological neoplastic disease is occurrence of uterine fibroids.

21. The agent for use according to any one of the preceding items, wherein the agent is formulated for subcutaneous or intravenous injection.

22. An agent as specified in any one of the preceding items.

23. An agent comprising

  a. a ligand capable of specifically binding to FAP;

b. a radioisotope label.

24. The agent according to item 23, wherein the radioisotope label is selected from the group comprising $^{94m}$Tc, $^{186}$Re, $^{203}$Pb, $^{47}$SC, $^{111}$In, $^{97}$Ru, $^{62}$CU, $^{88}$Y, $^{121}$Sn, $^{161}$Tb, $^{153}$Sm, $^{166}$Ho, $^{105}$Rh.

25. An agent according to item 23 or 24 for use in diagnosis of a benign gynecological neoplastic disease.

26. The agent for use according to item 25, wherein the benign gynecological neoplastic disease is endometriosis or uterine fibroids.

The invention is further illustrated by the following examples and figures, from which further embodiments and advantages can be drawn. These examples are meant to illustrate the invention but not to limit its scope.

*Description of the Figures*

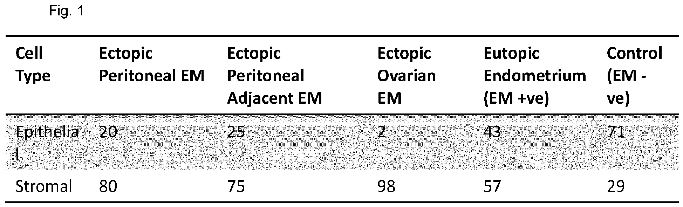

Fig. 1    shows single-cell RNA-seq showing unbiased cellular composition of samples of ectopic, eutopic and control endometrium. Fibroblasts are overrepresented in ectopic and eutopic endometrium from women with endometriosis and are generally much higher than healthy eutopic endometrium from controls. Data from Tan et al., 2021 (https://doi.org/10.1101/2021.07.28.453839), reanalyzed for this figure.

Fig. 2    shows the designs applied in this disclosure.

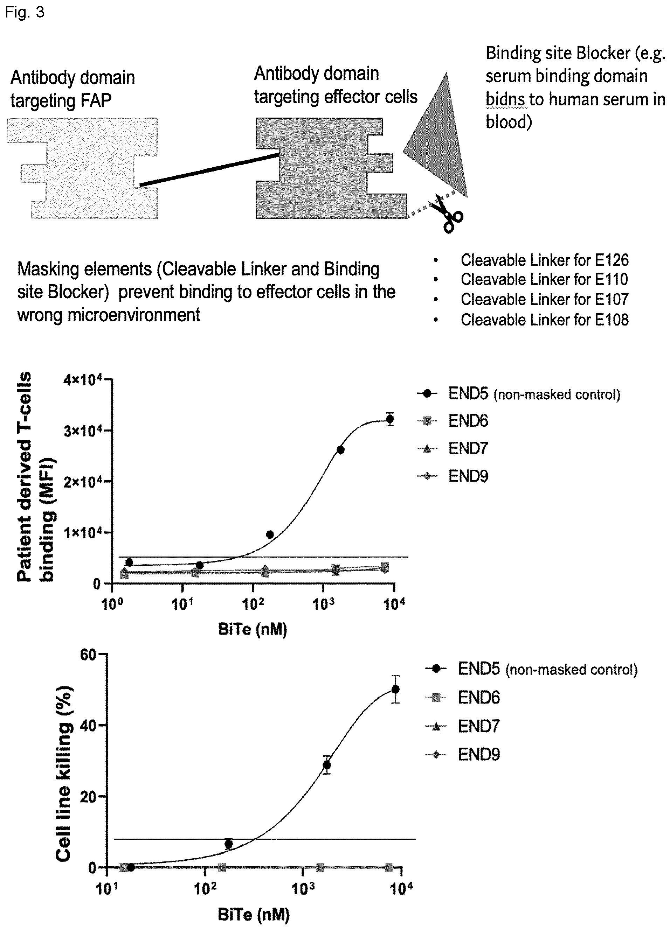

Fig. 3    shows a diagram showing an examplatory implementation of the masking approach protecting the immune cell binding domain. The plots beneath show the masking approach and that the activatable format of the treatments had no binding or cell-killing ability and could recover their binding and killing abilities upon cleavage and activation.

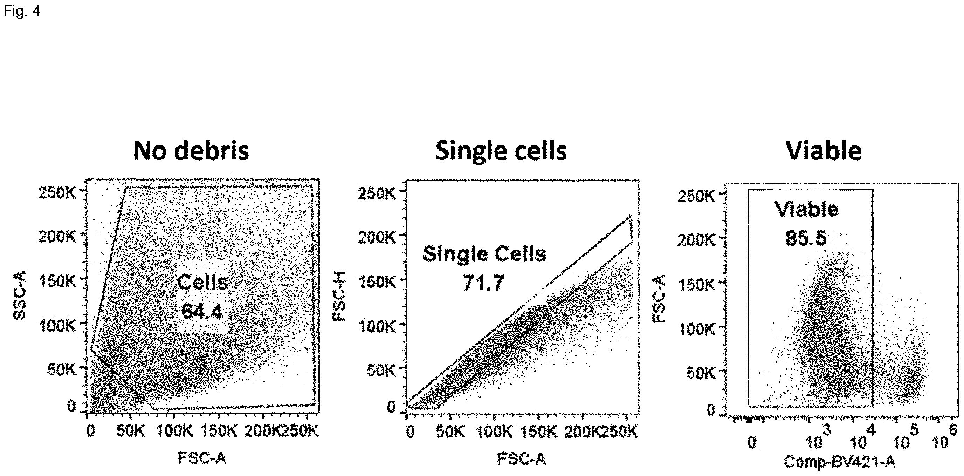

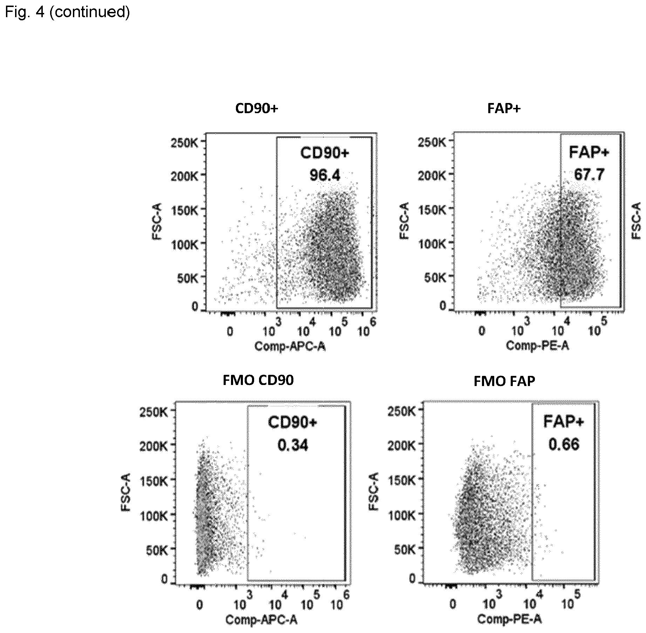

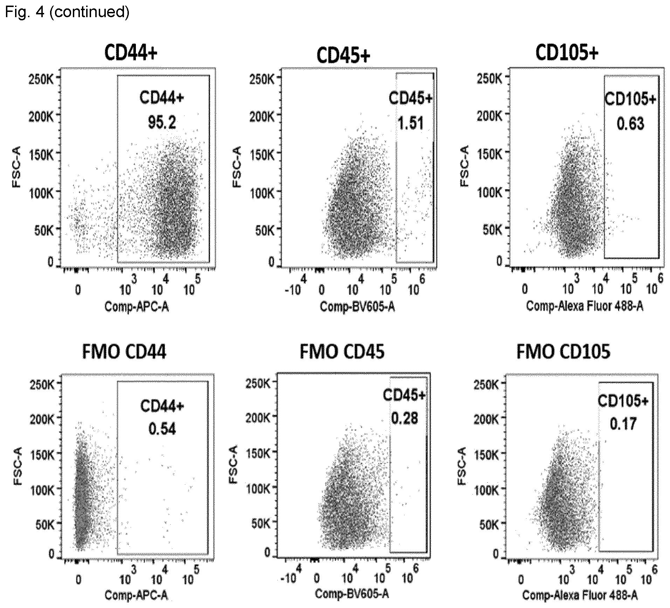

Fig. 4    shows FAP phenotyping panels. - PE569 ovarian endometrioma (OMA). PE569 cultured ovarian endometrioma stromal cells. PE569 OMA gating strategy is shown. CD90-APC, FAP-PE, L/D-ZombieV. CD44-APC, CD45-BV605, CD105-AF488. L/D-ZombieV.

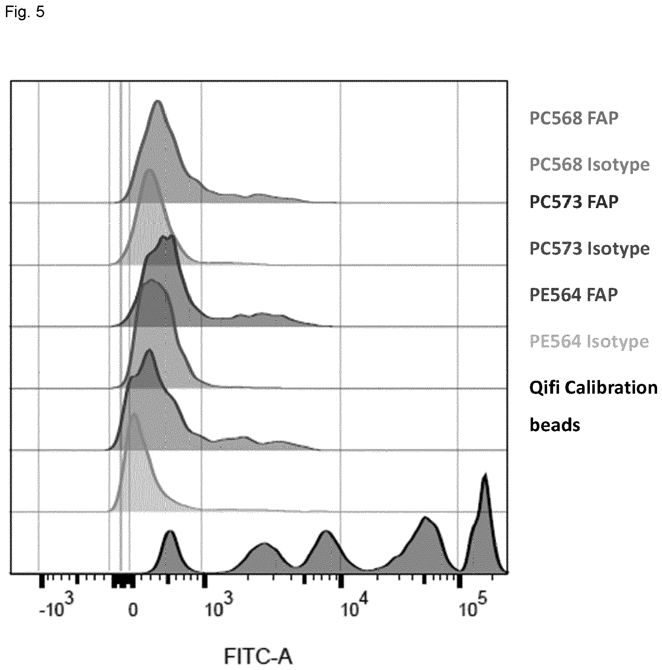

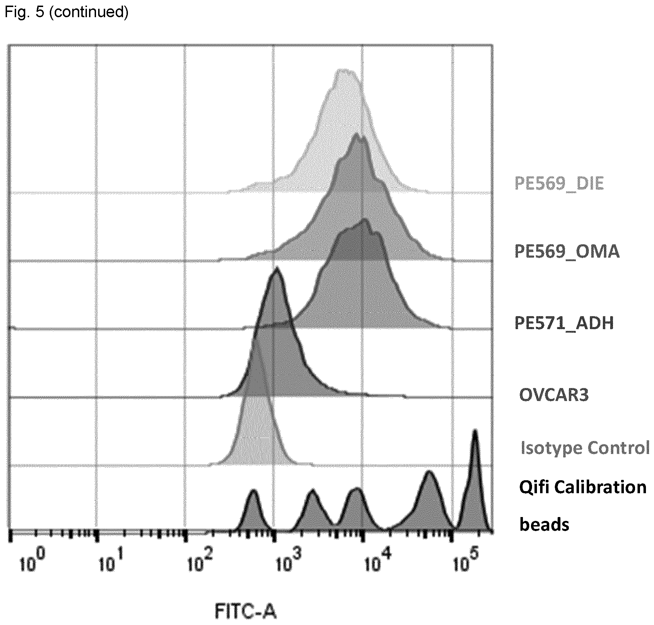

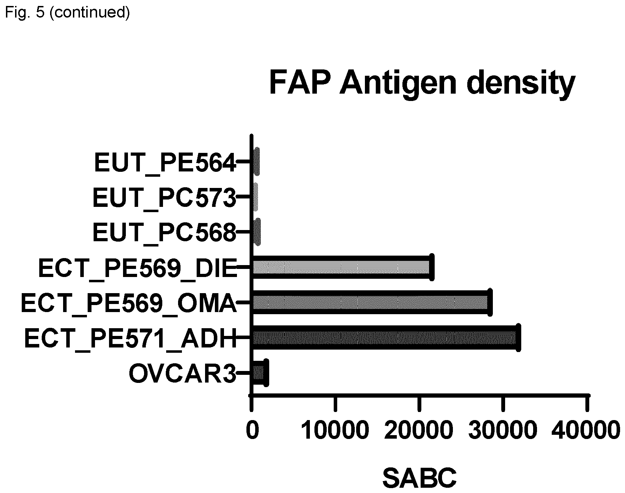

Fig. 5    shows FAP antigen density eutopic versus ectopic endometrial tissue. Qifikit was used for the quantitative determination of cell surface FAP antigen by flow cytometry using direct immunofluorescence assay. The antigen density is expressed as Specific Antibody-Binding Capacity (SABC) units. Samples analyzed from lesions (ectopic): PE569_DIE (deep infiltrating endometriosis), PE569_OMA (ovarian endometrioma), PE571_ADH (ovarian endometrioma adhesions), OVCAR-3 (human ovarian cancer cell line). Samples analyzed eutopic: PE564, PC573, PC568. Low surface expression was observed on eutopic endometrium.

Fig. 6    shows that average eutopic has 13 % FAP positive cells and that average ectopic has > 83 % FAP positive cells.

Fig. 7    shows evident FAP and CD90 expression on stromal cells compared to epithelial cells: MFI and expression level - Phenotyping PE575 and PE576.

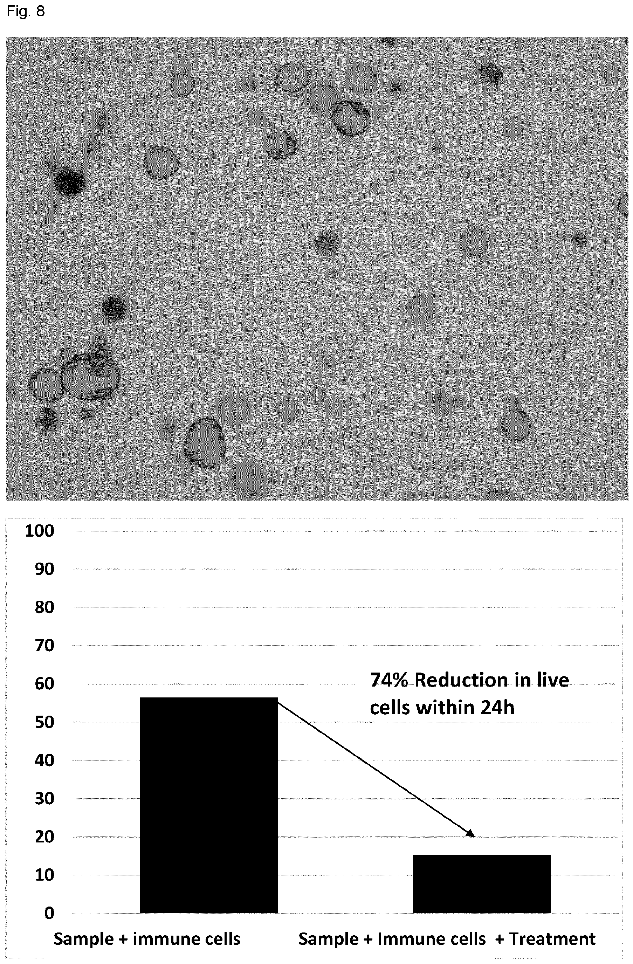

Fig. 8    shows *ex-vivo* killing assay: Patient derived organoids of lesions cocultured with immune cells and antibody for 24 hours.

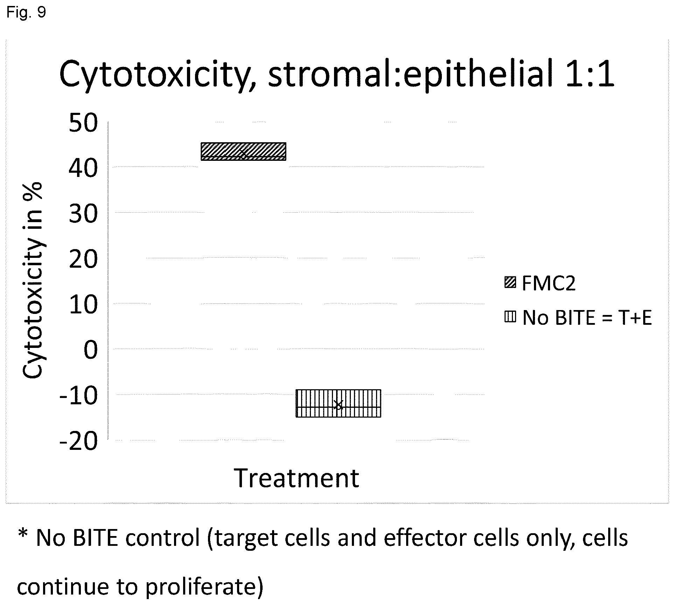

Fig. 9    shows killing assay CellTiterGlo PC571 with FAP BITE.

Fig. 10    shows killing assay by (brightfield) microscopy. Eutopic Endometrium cells-Passage 0 PC571.

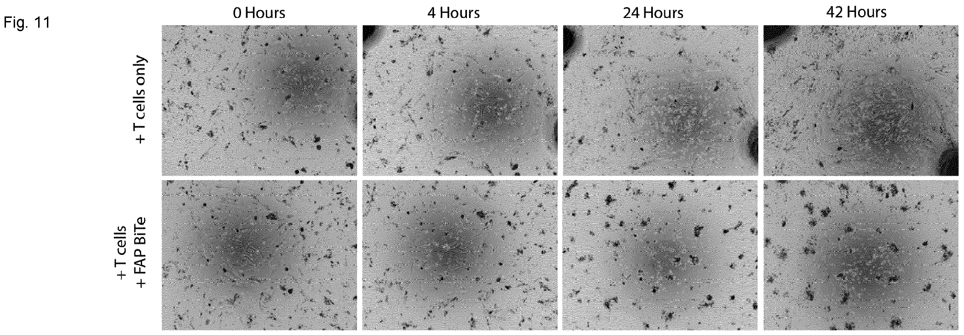

Fig. 11    Killing assay by (brightfield) microscopy. Primary cells from endometriosis tissue - Passage 1 PE571.

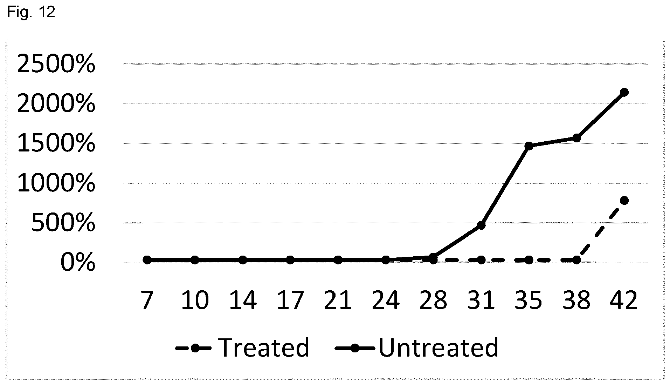

Fig. 12    shows OVCAR3 + T cells (Endometriosis patient) in NOG mice. *In-vivo* pilot in Mice: implant Growth delayed by 2 weeks. Mice were grafted with aggressive tumor cell-line expressing the target protein. Treatment was administered 6 × between d7 to d14. T cell were administered 2x (1xper week) Dose 0.1 mg/kg. Tumor volume was measured every 3 days.

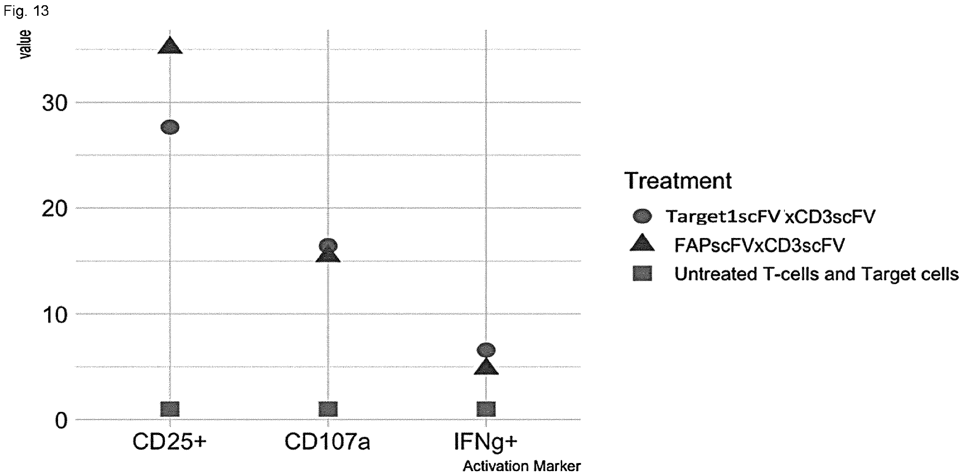

Fig. 13    shows immune response against patient samples indicates specific constructs activity against FAP+ endometriosis cells (mixed epithelial and stromal cells 1:1).

Fig. 14    Flow cytometry data showing the high proportion of FAP+ cells in uterine fibroids (myoma) patient lesions

Fig. 15    Serum level of MMP26 from women with endometriosis, myoma, and healthy

Examples

As part of a newly started clinical study (ethics approval number: BASEC 2020-02117), 62 women contributed samples to the inventors' study to date. The samples have been collected from women with endometriosis and women who are undergoing gynecological surgeries for non-endometriosis related interventions (e.g., myoma or tubal ligation). For women with endometriosis the following samples were collected: endometriotic lesions, endometriosis adhesions (when present), and eutopic endometrium. For the controls, eutopic endometrium and diseased tissue if present (e.g. myoma) is collected. For all women blood is collected and the samples are collected and processed according to WERF SOPs (to the extent possible). Two additional ethics approval (BASEC 2020-02272) and (BASEC- 2021-00885) were obtained for healthy buffy coats and in-vivo xenograft testing. The median age of the participants to date is 35 years old.

*Example 1: Target Validation*

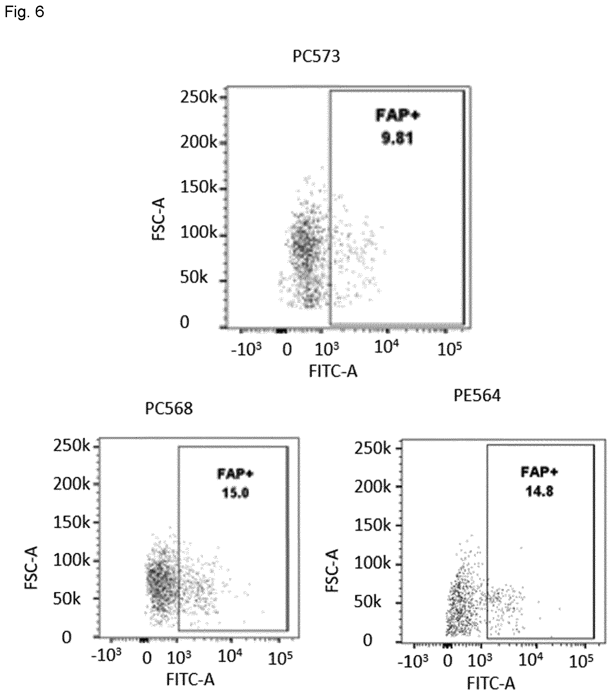

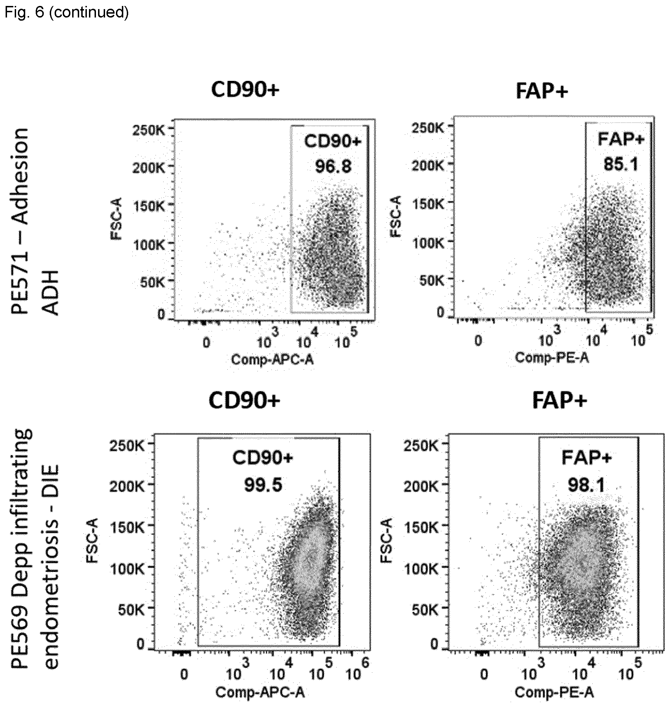

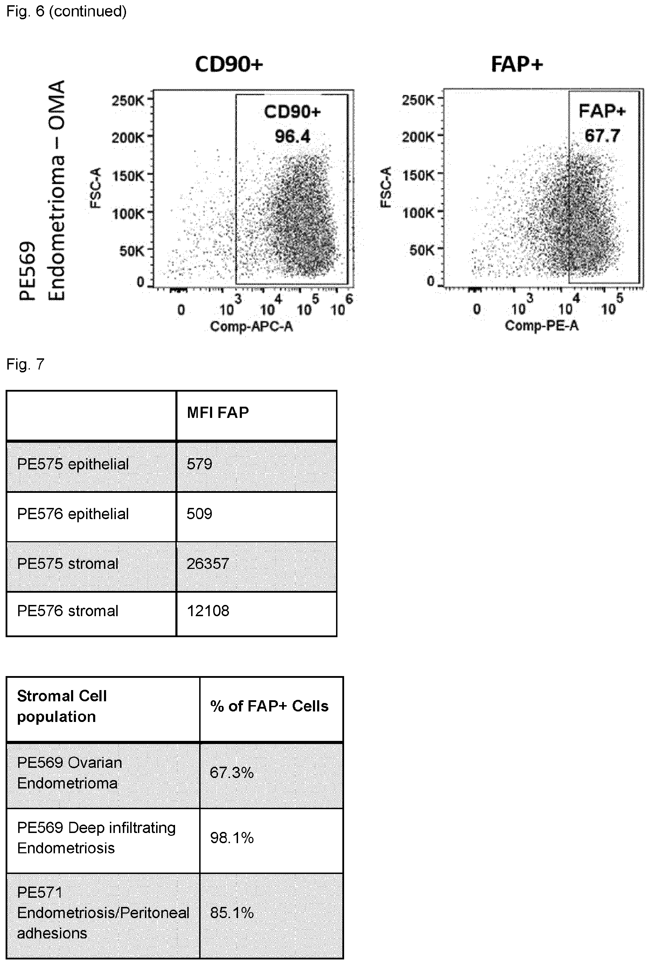

The inventors demonstrated for the first time, that in endometriosis patient samples FAP protein is expressed on the surface of ectopic endometrium as well as endometriosis associated adhesions. Depending on the sample, the proportion of FAP+ stromal cells was 67.3% for ovarian endometriosis, 98.1% for deep infiltrating endometriosis, and 85.1% for endometriosis adhesions. In comparison, the eutopic endometrial fibroblasts expressed FAP 13.8% on average. The epithelial cancer cell-line OVCAR3 was 13.60% positive for FAP, see phenotyping (Fig. 6). Using a two-sided t-test, ectopic endometrium stromal cells were found to have a significantly higher percentage of FAP+ cells.

Using immunohistochemistry, most healthy tissues have not shown any FAP expression (see supplementary material of (Schuberth et al., 2013, J Transl Med. 11: 187.), and at a very low level on the healthy endometrium on a subset of fibroblasts (see Fig. 5-6). Moreover, the inventors confirm the low expression of FAP in the healthy uterus. FAP's differential expression differentiates the tissue remodeling associated with the endometrial cycle from active ectopic tissue fibrosis. It also highlights the potential origin of the ectopic fibroblasts that may carry the FAP$^+$ phenotype from the eutopic endometrium to ectopic regions along with epithelial cells during retrograde menstruation.

The inventors then assessed the number of surface copies of FAP using flow cytometry (QIFIKIT). For the eutopic endometrium, ectopic endometrium, and adhesions. The surface copy number of FAP was 20-35 folds higher in the ectopic endometrium and adhesions compared to the eutopic endometrium from women with endometriosis. More specifically the copy number on the eutopic endometrium was <1000, while the ectopic endometrium and adhesions had between 25000-35000 surface copies. To put the numbers in perspective, healthy liver cells which show very low expression in immunohistochemistry have <1000 surface FAP copies. A similar difference in surface density has been noted in cancer immunotherapy where responders versus non responders had respectively >10000 vs <2000 copy number of the target antigen.

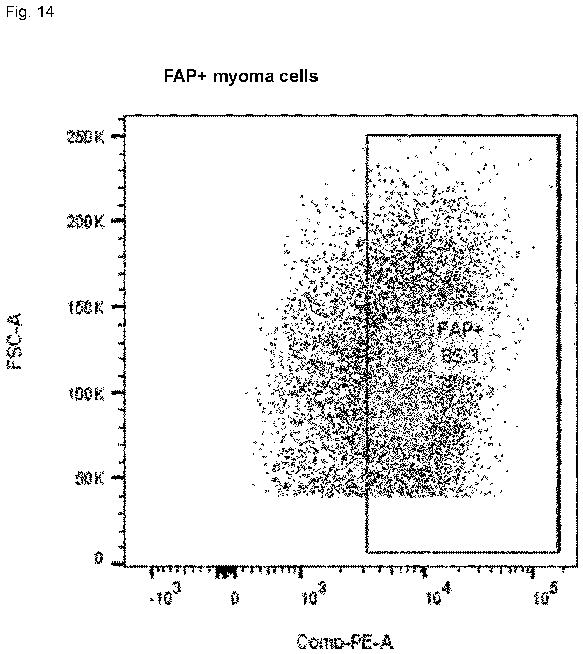

The inventors show also that Uterine fibroids, similar to endometriosis, are highly FAP positive (Fig. 14).

*Example 2: Design of a bi-specific antibody for the cytotoxic destruction of endometriosis FAP positive Fibroblasts*

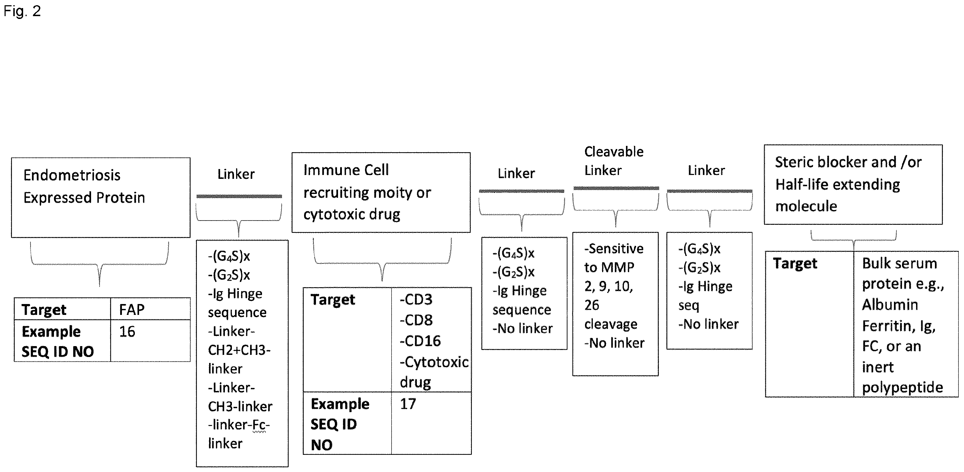

In order to target endometriosis overactivated fibroblasts that are FAP positive, the inventors engineered bi-specific antibody constructs. These constructs follow the design in Fig. 2, where FMC-2 is a bi-specific antibody targeting FAP. The design of FMC-2 with which the inventors have generated the data presented in this document is based on scFvFAP (-scFvCD3 (OKT3) from the public domain. The inventors have generated their own nanobodies (single domain antibodies) in alpacas for FAP, CD3, and CD16, with over 250 positive binders. The inventors' objective is to engineer 1-2 final lead designs that utilize the inventors' proprietary nanobodies and which are optimized for safety, efficacy, developability, and manufacturability.

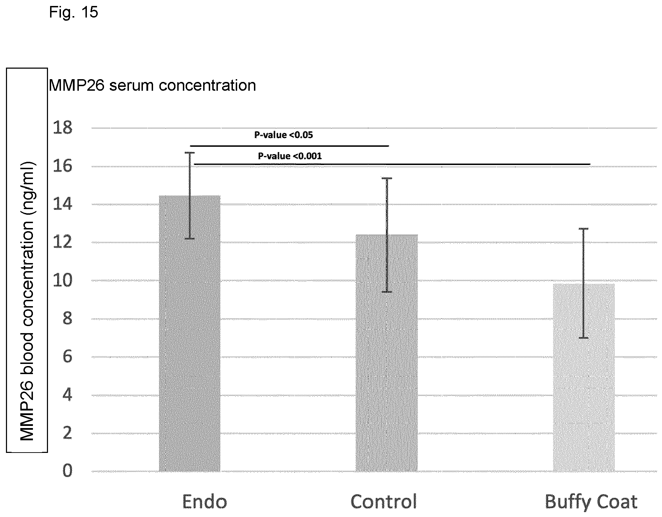

The first safety feature is a masking strategy with endometriosis specific cleavage. The basic principle is the introduction of a moiety that prevents one or more of the antibody domains from binding to its target until it meets the right (endometriotic) microenvironment. Briefly, the masked antibody during circulation in the body has no cytotoxic effects, and upon meeting the defined microenvironment, the linker sensitive to specific proteases is cleaved off along with the masking moiety, exposing the binding domain and activating the antibody. For endometriosis specifically, the inventors designed 4 different linkers that are sensitive to enzymes overexpressed in the endometriosis microenvironment. As a first example, the inventors demonstrated MMP26 overexpression in patient samples compared to myoma patients, and healthy donors (Fig. 15, p value <0.05 and <0.001 respectively). Another enzyme the inventors have tested linkers for is MMP2, which has been demonstrated to be overexpressed, and specifically over 9 folds overexpressed in endometriosis fibroblasts compared to control eutopic fibroblasts (p-value <0.001 under Bonferroni correction, reanalysis of GSE168902 using standard R limma pipeline). MMP2 is also known to contribute to fibrosis and is one of FAP's protein interaction partners (String-db, v11.5).

*Example 3: Discussion*

Current targeting approaches of FAP are largely through small molecules, but these are not always specific to FAP (due to 52% homology with another protein), and small molecules are prone to cyclisation and inactivation (Fang J et al. Int J Cancer. 2016;138(4):1013-1023. doi:10.1002/ijc.29831). The inventors' unique approach is to target the protein's ecto domain using single domain antibodies to leverage their ability of accessing epitopes less accessible to Ig like antibodies. The inventors are starting the selection from >100 strong binders the inventors generated in alpacas for FAP. FAP shares up to 62% of its sequence with other known enzymes and it is hypothesised that previous inefficacies observed in cancer might be attributed to cross reactivity and the non-catalytic activity of FAP. The inventors' nanobodies program and selection approach shall avoid nonspecific binding and optimise for safety of the antibody as well as ensure specificity towards active fibrosis/adhesions.

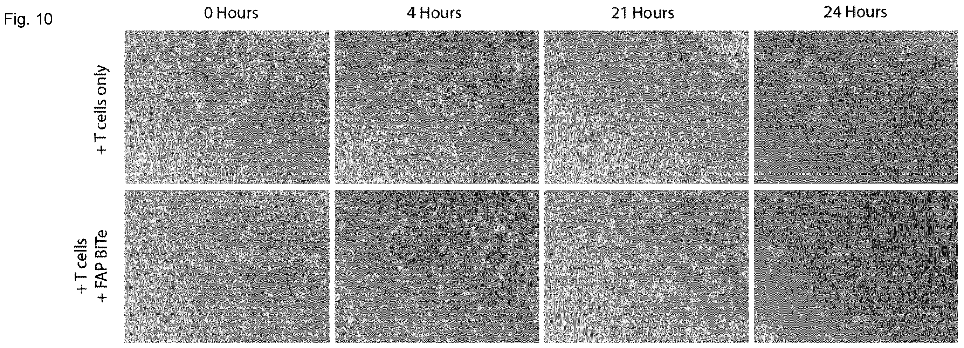

FMC-2 is the inventors' bi-specific antibodies program targeting FAP. Current generation FMC-2 (scFvFAP-scFvCD3) showed high efficacy in destroying patient derived stromal cells, which were more than 90% FAP+ (>90% destruction in 12h, 1:1 E:T ratio). Compared to endometriotic lesions, >80% of the eutopic endometrium fibroblasts survived the treatment with FMC2 after 24h under the same conditions (Fig. 10). FMC-2 was also extremely effective in destroying endometriosis related adhesions, clearing the adhesion cells in less than 6h (1:1 E:T ratio). While surgeries have been shown to be efficient in pain relief (despite high recurrence rates), they are also a main contributor in peritoneal adhesions.

FMC-2 has demonstrated strong efficacy against endometriosis patients derived stromal cells and adhesions. FMC-2 holds extraordinary potential to reduce the necessity of surgeries for women with endometriosis related fibrosis and adhesions, and potentially delay hysterectomies. Additionally, FMC-2 may be applicable to FAP+ heart fibrosis and general surgery related peritoneal adhesions which occur at up to 93% of surgeries.

*Example 4: Material and Methods*

*Protocol for single purified cell suspensions*

Colagenase I/II digestion mix:

|  | Stock | Endconcentration |
|---|---|---|
| **HBSS with Mg2+/Ca2+** | 1x | 1x |
| **Colagenase I** | 150 mg/ml | 2 mg/ml |
| **Colagenase II** | 80 mg/ml | 0.8 mg/ml |
| **DNAse I** | 2 mg/ml | 40 ug/ml |
| **Rock Inhibitor Y-27632 optional** |  | 10 uM |

- Estimate amount in ml needed to digest tissue. Use 3 ml of digestion mix per lesion
- Weight of biopsy tissue
- Gently shake to remove excess blood and mucus.
- Centrifuge 300 g for 5 min
- Wash with PBS (without $Mg^{2+}/Ca^{2+}$) 1 × for endometriosis.
- Centrifuge at 300 × g for 5 min
- To spare tissue for fixation, freezing etc cut the initial pieces without media and move to retrospective tubes.
- Decant the biopsy into a Petri dish containing 3 mL of digestion media. Use 6 ml for myoma. Add digestion media on the tissue for digestion:
- Mince the fresh tissue with forceps and scissors into 1-mm$^3$ small pieces in digestion media.
- Use 25 ml stripette to transfer the pieces and digestion media into gentleMACS flask (C tube).
- Rinse the mincing dish with 1-5 mL of digestion media and add to the gentleMACS flask.
- Incubate on gentleMacs on program for 40 min at 37°C. After gentleMACS program ends, do NOT open flask until centrifugation.

- Centrifuge the sample at room temperature at 300 $\times$ g for 5 min.
- Gently remove digestion media by pipette or stripette
- Wash in PBS without $Mg^{2+}/Ca^{2+}$ (Myoma until supernatant becomes clear)
- Centrifuge at 300 g for 5 min, remove supernatant
- Resuspend in Tryple 1-3 ml and Rock Inhibitor(10 ul per 1 ml), transfer to fresh 15 ml tube
- Incubate at 37°C for 15 min in shaker at 37°C
- Stop digestion by adding 14 ml PBS
- Wash in PBS (without $Mg^{2+}/Ca^{2+}$) 1 $\times$
- Centrifuge at 300 g for 5 min
- Remove supernatant
- Add 5 ml PBS (without $Mg^{2+}/Ca^{2+}$)
- Filter the digestion through a 70-mm cell strainer.

   ○ Collect flow-through fraction,
   ○ Collect backwash fraction with 5 ml culture media, plate on dish

- Centrifuge 300 g for 5 min
- Resuspend in 1- 10 ml PBS (without $Mg^{2+}/Ca^{2+}$) and count viable cells (trypan blue)
- Centrifuge at 300 g for 5 min
- Add ACK Lysing buffer 1-3 ml
- Incubate for 8 min at RT
- Stop the reaction by filling the tube with PBS (15 ml)
- Centrifuge at 300 g for 5 min
- Wash with PBS without $Mg^{2+}/Ca^{2+}$ 1$\times$.
- Pellet can be reconstituted or frozen.

*Organoid culture media*

Material

- DMEM F-12 (+ L-Glutamine, + Hepes, + Phenol Red); Gibco™ ; #11330032
- B27 Supplement 10ml; Gibco™; #12587-010 (stock 50X; -20C)
- N-2 Supplement (100X)-5 mL; Gibco™; #17502048 (stock 100X; -20C)
- A -83-01; Sigma Aldrich; #SML0788-5MG (stock 5000X-2500uM; -20C)
- SB -202190; Sigma Aldrich; #S7067-5MG (stock 2500X-25000uM; -20C)
- Recombinant Human EGF, CF; Bio-Techne AG; #236-EG-01M (stock 100ug/mL; - 80C)
- Recombinant Human Noggin; Peprotech; #120-10C-20uG (stock 200ug/mL; -80C)
- Recombinant Human FGF basic (146 aa); Bio-Techne AG; #233-FB-025 (stock 4ug/mL; -80c)
- Penicillin-Streptomycin; Sigma Aldrich; #P4333-100ML (stock 100X; -20C)
- INSULIN-TRANS-SEL-G, 100X; Gibco™; #41400045 (stock 100X; 4C)
- GlutaMAX Supplement-100 mL; Gibco™; #35050061 (stock 100X; 4C)
- Human FGF-10; Pepro Tech EC Ltd; #100-26-100uG (stock 25mg/mL; -20C)
- B-ESTRADIOL; Sigma Aldrich; #E8876-1G (stock 10mg/mL; 4c)
- N-ACETYL-L-CYSTEINE -SIGMA -GRADE; Sigma Aldrich; #A7250-50mg (stock 100ug/mL)
- Y-27632 dihydrochloride (100MG); AbMole BioScience; #M1817 (stock 1mM; -20C)
- RSPO1-conditioned medium; Homemade (5%; -20C)
- NICOTINAMIDE; Sigma Aldrich; #72340-100G (stock 1M; -20C)
- Recombinant Human HGF; Peprotech; #100-39-100UG (stock 100ug/mL; -20C) Procedure
- Thawing on ice the reagents
- Prepare the media on ice according to Boretto et a., 2019: Patient-derived organoids from endometrial disease capture clinical heterogeneity and are amenable to drug screening
- Use the media within 1 week
- Filter the media before using (0.22um)
- Use for 50mL:

   DMEM F-12: 41.1mL

B27 Supplement: 1ml

N-2 Supplement: 500ul

A -83-01: 10ul

SB -202190: 20ul

Recombinant Human EGF, CF: 25ul

Recombinant Human Noggin: 20ul

Recombinant Human FGF basic (146 aa): 25ul

Penicillin-Streptomycin: 500ul

INSULIN-TRANS-SEL-G: 500ul

GlutaMAX Supplement: 500ul

Human FGF-10: 20ul

B-ESTRADIOL: 20ul

N-ACETYL-L-CYSTEINE: 102ul

Y-27632 dihydrochloride: 500ul

RSPO1-conditioned medium: 5mL

NICOTINAMIDE: 100ul

Recombinant Human HGF: 50ul

*Organoid harvesting procedure: Cultrex™ Organoid Harvesting Solution*

Materials (according to manufacturers guidelines)

- Cultrex™ Organoid Harvesting Solution; #3700-100-01 (100 mL)
- PBS1X cold
- Tryple Express; #12604-013
- Y-27632 dihydrochloride (100MG); AbMole BioScience; #M1817 (stock 1mM)
- Cultrex Reduced Growth Factor Basement Membrane Extract, Type 2, Pathclear; Biotechne; # 3533-005-02

Procedure

- Working on ice, aspirate cell culture media and gently wash each well with 10 volumes of cold (2-8 °C) PBS. Be careful not to disrupt basement membrane matrix containing organoids.
- Aspirate the PBS, and add 10 volumes of cold (2-8 °C) Cultrex™ Organoid Harvesting Solution to each well.
- Incubate the plate at 2-8 °C or on ice for 30-90 minutes with moderate shaking. This incubation is complete when the basement membrane matrix dome is no longer visible at the bottom of the well and the organoids are seen floating at the bottom of the well. Note: Dislodging the dome with a cell scraper or pipet may accelerate this process.
- Once the matrix depolymerizes, transfer contents of the well into a tube on ice. Single wells may be transferred to a microtube while multiple domes may necessitate a 15 mL or 50 mL conical tube.

- Centrifuge the tube at 500 × g for 5 minutes at 2-8 °C in a swinging bucket rotor to pellet the organoids. Aspirate the supernatant.
- Wash organoids with 10 volumes of cold (2-8 °C) PBS, and repeat centrifugation at 500 × g for 5 minutes at 2-8 °C in a swinging bucket rotor to pellet the organoids. Aspirate the PBS.
- Add 1mL of Tryple with 10ul of Rock inhibitor and incubate for 15min at 37
- Add 10volumes of PBS to neutralize the Tryple with Rock inhibitor
- Centrifuge the tube at 500 × g for 5 minutes at RT. Aspirate the PBS
- Isolated organoids may be:

  ○ Resuspended in basement membrane matrix for further organoid culture.
  ○ Resuspended in freezing medium for cryopreservation.
  ○ Killing assay

*Phenotyping of eutopic and ectopic endometrial cells isolated from tissue*

Phenotyping of eutopic and ectopic endometrial cells with the established panels. Panel 1 assesses the ratio of epithelial and stromal fraction, while Panel 2 is used to further characterise the stromal fraction. Together with the Phenotyping, Quantification of FAP is performed using the Qifikit.

Materials

• Eutopic and ectopic cells
• OVCAR3 cells
• PBS (Gibco, phosphate buffered saline, w/o CaCl2 or MgCl2, Ref 10010-015, Lot 2375270)
• FBS (Gibco, Cat 10500064, Lot 229262H)
• NaN3 (Sigma-aldrich, sodium azide, S2002-100g, Lot STBJ9889)
• VersaComp Antibody Capture Bead Kit (Beckmancoulter, ref B22804, lot 4132061 K)
• ArC Amine Reactive Compensation Bead Kit (Invitrogen, ref A10628, lot 2409708)
• Fc Block (Human TruStain, Biolegend, Cat 422302, Lot B328706)
• Antibodies:

| Marker | Fluorophore | Provider | Cat No | Concentration | Final Conc. (2x) |
|---|---|---|---|---|---|
| CD90 | AF-647 | BioLegend | 328116 | 1:80 | 1:40 |
| FAP | PE | R&D systems | FAB3715P | 1:40 | 1:20 |
| Zombie Violet | L/D | BioLegend | 423114 | 1:100 | 1:50 |
| CD45 | BV605 | BioLegend | 304041 | 1:40 | 1:20 |
| CD105 | AF488 | BioLegend | 323209 | 1:40 | 1:20 |
| CD44 | APC | BioLegend | 397505 | 1:40 | 1:20 |

Procedure

- Collect cells with Trypsin/Accutase, count and seed into 96-well u-bottom plates at 100 ul per well in PBS (50-100k per well).
- Prepare FACS Buffer: PBS + 2% FBS + 0.1% NaN$_3$.
- Add 100 μl of FB per well, resuspend and spin for 5 min at 300 xg at 4°C. Repeat the wash with 180 μl of FB.
- Incubate 15 min at RT with 50 μl of BD human Fc block at 1:100 for 1-10×10$^6$ cells in PBS.
- While incubating, prepare the phenotyping panel. Make sure to add the L/D stain at the very end. Add 50 μl directly labelled antibody dilution to each well.
- For compensation add one drop of VersaComp compensation capture beads to each well (use ArC reactive beads for L/D). Make sure to vortex the beads for at least 30 sec before adding. Place single colour antibody conjugates into the corresponding wells at the antibody concentration used for your application.
- Incubate for 40 min in the dark at 4°C.

- Top up with 150 μl of FB (PBS for compensation beads) and spin for 5 min at 300 xg, discard the supernatant.
- Wash once with 180 μl of FB.
- Resuspend the cells in 200 μl of PBS + 2mM EDTA.
- Store the plate at 4°C under light protection until measurement on the Celesta. Before measurement transfer the content of the plate to labelled FACS tubes.
- Do not forget to add one drop of the corresponding negative beads (Component B) to each compensation tube and vortex before acquisition on the flow cytometer.
- Panel 1: CD90-AF647, FAP-PE, L/D-Bv421
- Panel 2: CD45-BV605, CD105-AF488, CD44-APC, L/D-BV421

*FAP Quantification with Qifikit (according to manufacturer's recommendations)*

Qifikit contains a series of beads, 10 um in diameter and coated with different, but well-defined quantities of mouse Mab molecules. The beads mimic cells with different antigen densities which have been labelled with a primary mouse Mab, isotype IgG. Cells are labelled with primary mouse monoclonal antibody directed against the antigen of interest. In a separate test tube, cells are labelled with irrelevant mouse monoclonal antibody (control).

Then, cells, set-up beads and calibration beads of the kit are labelled, in parallel, with fluorescein-conjugated anti-mouse secondary antibody.

The primary antibody used for labelling of the cells is used at saturating concentration. The saturation conditions are determined by performing titration studies on each Mab investigated using the FITC-conjugated anti-mouse secondary antibody. The primary antibody may be of any mouse IgG isotype. Under these conditions, the number of bound primary antibody molecules corresponds to the number of antigenic sites present on the cell surface. The secondary antibody is also used at saturating concentration. Consequently, the fluorescence is correlated with the number of bound primary antibody molecules on the cells and on the beads.

Materials

- Qifikit (Dako, K0078) with Set-Up beads (vial 1), Calibration beads (vial 2), FITC conjugate (vial 3) .
- FAP unconjugated primary antibody (FAP Monoclonal Antibdoy, eBioscience, Invitrogen, BMS168), 1 mg/ml
- Unconjugated IgG1 primary antibody (BioLegend, Cat No 401402), 0.5 mg/ml
- FACS buffer (PBS + FBS + NaN3)
- 100k for FAP Quant & Full Panels, and 80k for all the FMOs.

Procedure

- Mix 100 μl cell suspension with 10 μl of an unconjugated FAP primary mouse monoclonal antibody (1:200). For the negative control, replace the unconjugated antibody with an irrelevant IgG1 mouse monoclonal antibody of the same isotype and adjusted to the same concentration. Ensure that the primary antibody is used at saturating concentration.
- Incubate at 4°C for 45 min.
- Add 150 μl of FACS buffer. Mix gently to ensure that the cells are in suspension.
- Centrifuge at 300 xg for 5 min. Asipirate and discard the supernatant, leaving approximately 50 μl of fluid in the well.
- Repeat the wash with 180 μl of FACS buffer.
- Prepare the Set-Up beads and Calibration beads: Place 100 μl (50 ul might also be enough) of vortexed beads from vial 1 and vial 2, respectively, in two separate wells. Add 150 μl of PBS-BSA, resuspend well and centrifuge at 300 × g for 5 min. Aspirate supernatant.
- Add 100 μl of Vial 3, FITC conjugate, diluted 1:50 in PBS (add to the cell suspension as well as the Set-Up and Calibration beads). Mix gently to ensure that the cells are in suspension.
- Incubate in the dark at 4°C for 45 min.
- Top up with 150 μl of FACS buffer. And centrifuge at 300 xg for 5 min.
- Aspirate and discard the supernatant. Repeat the wash with 180 μl of FACS buffer.
- Resuspend the pellet in 200 μl of FACS buffer. Store test tubes at 4°C for no more than 2 hours before analysis.

Data Acquisition

- Set up the flow cytometer using standard operating procedures.
- First establish the window of analysis. Since the autofluorescence of the Qifikit beads tends to be higher than that of lymphocytes, it might be necessary to make pre-adjustments of the PMT of the corresponding fluorescence detector to assure that both negative cells and populations of the Set-Up Beads are displayed on scale.
- Acquire the data from the Set-Up beads. Fluorescence analysis is confined to beads singlets clear of debris, as defined on a forward scatter versus side scatter dot plot.
- Without chaining the Window of Analysis, acquire the data from the calibration beads and the sample.
  → Data from beads and cell specimens have to be collected at the same time. PMT voltage from the corresponding fluorescence detector has to be the same, whereas scatter settings may vary from cell to Qifikit bead data acquisition.
- Samples are analysed in the following order:

  → Vial 1: Set-Up beads. This sample is used to establish the Window of Analysis. The Set-Up Beads comprise a mixture of bland beads and high level-beads.

  → Vial 2: Calibration beads: this sample is used for the construction of the calibration curve (MFI against ABC)

  → Cells are analysed on the flow cytometer and ABD is calculated based on the equation of the calibration curve.

*Killing assay with CellTiter-Glo®*

Prepare the CellTiter-Glo® Reagent according to the manufacturer's guidelines Material

- T cell donor: expanded T cells from PE551 donor
- Target cells: P1 PC571, stromal + epithelial fraction mixed 1:1
- Cell culture medium used for cell seeding: DMEM/F12 medium (Gibco, Ref 11330-032) + 10% FBS + 1% P/S
- Trypsin 0.25%, phenol red (Product number: 25200056)
- Assay medium: RPMI no phenol red (10%FBS and 1X L-Glut)
- 96-well black plate with clear bottom (Product number: 353219)
- BITEs:FAP BITE,
- CellTiter Glo viability assay kit
- Procedure
- Cells at P0 were detached by Trypsin for the stromal fraction and Accutase for the epithelial fraction for approx. 5 min and 15 min for each cell fraction, respectively. Cells were collected, centrifuged at 300 × g for 5 min, and the two cell fractions were mixed and used for next experiments. For this assay, 7000 cells/ well (in 100 ul) were seeded in DMEM/F12 + 10% FBS + 1% P/S culture medium, and left to adhere overnight.
- The next day, the cell culture medium was replaced with T-cell culture medium (only 50 ul cell culture medium was added), and in each well according to the layout below, T-cells (28 000/well) (1:4 ratio of target cells: T cells) and or/Bites in a total volume of 25 ul (final concentration of 1200 pg/mL).
- Lysis buffer was added in some of the wells as control condition for cell killing, and it was added 30 min before addition of CellTiter Glo reagent. 4 uL of Lysis Buffer were added in each well where needed.
- CellTiter Glo was determined after 24h: CellTiter Glo reagent aliquot was thawed and protected from light. At the respective time point, in this case 24 hours post addition of T-cells +/- Bites, 100 uL (or 104 uL in the wells that had lysis buffer) of Celltiter Glo reagent were added per well, and the plate was incubated for 20 min in the incubator 37C, protected from light. After 20 min incubation, the luminescence signal was measured with Tecan plate reader.
- Record luminescence.
- Calculate the percent cytotoxicity as follows

  ○

$$[T - (TEAb - E)] / [(T - (Tdead - E)] \times 100$$

  ○ T = Target luminescent signal

○ E = effector luminescent signal,

○ Tdead = luminescent signal of target cells lysed

○ TEAb = Target + Effector + Antibody luminescent signal

*Killing assay - Microscopy (brightfield) Tracking*

Material

- Target cells PC5711 donor (passage 0), 25k epithelial + 25 k stromal (in 24 well plate)

- PE571 donor (passage 1) - total 50k cells (in 96 well plate)

- T cell donor: expanded T cells from PE551 donor

- BITEs:FAP BITE

- Assay medium: RPMI no phenol red (10%FBS and 1X L-Glut)

Procedure

- T cell: target cell ratio used 1:1

    ○ 24 well plate: 1:1. 50 k T cells seeded in each well in 300 uL (total volume per well was 400 uL of Tcell + BiTE)

    ○ 96 well plate: 10 k T cells seeded in each well, total volume 100 uL (total volume per well was 50 uL of Tcell + BiTE)

- Images of each well were taken at different timepoints: 0h, 2h, 4h, 21h and 24h

*Killing* assay *by flow cytometry*

Target cells (OVCAR3 or PE559-2) stained with CellTrace Yellow (5 uM) for 30 minutes. Then washed and cocultured with T cells at a ratio of 4:1 (E:T) for 24 hours. At the end of the incubation, cells are washed, fixed and acquired on Fortessa.

*Bispecific T cell engager (BiTe) staining protocol*

Material

- BiTe of interest

- Biotinylated mouse anti-Fab (https://www.iacksonimmuno.com Cat 115-066-006)

- Streptavidin PE (Biolegend Cat: 405203)

- Staining buffer

Procedure

- Distribute 100k target cells per well (96 u bottom) and centrifuge 500g for 5 minutes. (Do not forget to include an extra well for secondary control for each cell type)

- Remove the supernatant, wash the cells with 200ul of staining buffer, and repeat the same centrifugation step.

- Remove the supernatant and resuspend the cells in 100ul of staining buffer. Include 5ul (or relevant amount for your titration) of BiTe to the relevant wells (can prepare a mixture and resuspend the cells with that)

- Incubate the cells for 1 hour at 4C.

- Centrifuge at 300g for 5 minutes, remove supernatant, wash with 200ul of staining buffer, repeat centrifuge and remove supernatant.

- From this point onwards, secondary control conditions are also included in all the stainings.

- Prepare a mouse anti-Fab (1:200) mixture with staining buffer; e.g. 5 wells (consider a little extra) 550ul of staining buffer + 2.75 mouse anti-Fab. Resuspend the cells in 100ul of this mix.

- Incubate the cells for 30 minutes at 4C.

- Centrifuge at 300g for 5 minutes, remove supernatant, wash with 200ul of staining buffer, repeat centrifuge and remove supernatant.

- Prepare a Streptavidin-PE (1:200) mixture with staining buffer; e.g. 5 wells (consider a little extra) 550ul of staining buffer + 2.75 ul Streptavidin-PE. Resuspend the cells in 100ul of this mix.

- Incubate the cells for 30 minutes at 4C.

- Centrifuge at 300g for 5 minutes, remove supernatant, wash with 200ul of staining buffer, repeat centrifuge and remove supernatant.

- Resuspend the cells in 150ul of staining buffer and transfer each well to a flow tube.

- Acquire the cells.

*Assays with Masked BITEs*

- Masked BITEs were resuspended in the presence/absence +/- HAS (100ug/ml) in the staining buffer. T cells were added and incubated for 1 hour at 4C.

- Protease digestion:2ug BiTe was incubated with PBS or HBSS with Ca and Mg and Proteases for 3 hours at 37C in 50ul. 25k T cells were added directly in the mix (1 hour, 4C). Follow the BITE staining protocol.

**Claims**

**1.** An agent comprising

    a. a ligand capable of specifically binding to FAP; and
    b. a therapeutic moiety, wherein the therapeutic moiety is capable of triggering cell death of cells expressing FAP on their cell surface;

for use in treatment of a benign gynecological neoplastic disease.

**2.** The agent for use according to claim 1, wherein the therapeutic moiety is an immune cell recruiting moiety, wherein the immune-cell recruiting moiety is capable of specifically binding to an immune cell surface molecule.

3. The agent for use according to claim 2, wherein the immune cell surface molecule is selected from the group comprised of:

    a. CD3;
    b. CD16;
    c. CD8;

particularly wherein the immune cell surface molecule is CD3.

4. The agent for use according to any one of the preceding claims 2 or 3, wherein the immune-cell recruiting moiety is selected from the group comprising an antibody, an antibody fragment, a single-chain antigen-binding fragment, single-domain antibody, an aptamer, a non-immunoglobin scaffold, and an antibody-like molecule, particularly wherein the immune-cell recruiting moiety is selected from the group comprising an antibody, an antibody fragment, a single-chain antigen-binding fragment,
more particularly wherein the immune-cell recruiting moiety is a single-domain antibody.

5. The agent for use according to claim 1, wherein the therapeutic moiety is a cytotoxic molecule of molecular weight of <1000 Daltons (Da), particularly of molecular weight ≤800Da, more particularly of molecular weight ≤500Da, particularly wherein the cytotoxic molecule is selected from the group comprising doxorubicin, carminomycin, daunorubicin, aminopterin, methotrexate, methopterin, dichloro-methotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, leurosine, taxol, desacetylvinblastine, cyclophosphamide, ifosfamide, cytarabine, 6-thioguanine, chlorambucil, carmustine, mitoxantrone, paclitaxel or a cytotoxic derivative thereof.

6. The agent for use according to any one of the preceding claims, wherein the ligand is selected from the group comprising an antibody, an antibody fragment, a single-chain antigen-binding fragment, single-domain antibody, an aptamer, a non-immunoglobin scaffold, and an antibody-like molecule, a small molecule pharmaceutical obeying the Lipinski Rules of Five set of criteria, a peptide, and a FAP specific substrate, particularly wherein the ligand is selected from the group comprising an antibody, an antibody fragment, a single-chain antigen-binding fragment, more particularly wherein the ligand is a single-domain antibody.

7. The agent for use according to any one of the preceding claims, wherein the agent comprises a masking moiety which is attached via a linker to the agent, wherein the masking moiety is capable of reducing or eliminating the binding ability of the ligand and/or the immune cell recruiting moiety, particularly wherein the linker comprises a protease cleavage site.

8. The agent for use according to any one of the preceding claims, wherein the agent comprises a bulk protein binding site, particularly a human serum albumin binding site, wherein the binding site is attached via a linker to the agent, particularly wherein the linker comprises a protease cleavage site, particularly wherein the protease cleavage site is specifically cleavable by matrix metalloproteinase 2, 9, 10, and/or 26.

9. The agent for use according to any one of the preceding claims, wherein the endometriotic tissue is **characterized by** detectable expression of FAP, particularly wherein at least 1% of endometriotic cells express FAP.

10. An agent for use in treatment of a benign gynecological neoplastic disease, wherein the agent comprises

    a. a target protein binding moiety;
    b. an immune cell recruiting moiety;
    c. a masking moiety capable of shielding the immune cell recruiting moiety and/or the target protein binding moiety from binding to its target;
    d. a cleavable linker connecting the masking moiety with the other components of the agent, wherein the cleavable linker is specifically cleavable by matrix metalloproteinases (MMPs) 2, 9, 10, and/or 26, particularly wherein the cleavable linker is specifically cleavable by MMP 2 or MMP 9.

11. The agent for use according to claim 10, wherein the cleavable linker is **characterized by** a sequence selected from the group of SEQ ID NO 1 to SEQ ID NO 4.

12. An agent comprising

   a. a ligand capable of specifically binding to FAP;
   b. a radioisotope label;

   for use in diagnosis of a benign gynecological neoplastic disease, wherein the radioisotope label is selected from the group comprising $^{94m}Tc$, $^{186}Re$, $^{203}Pb$, $^{47}Sc$, $^{111}In$, $^{97}Ru$, $^{62}CU$, $^{88}Y$, $^{121}Sn$, $^{161}Tb$, $^{153}Sm$, $^{166}Ho$, $^{105}Rh$.

13. The agent for use according to any one of the preceding claims, wherein the benign gynecological neoplastic disease is endometriosis.

14. The agent for use according to any one of the preceding claims 1 to 12, wherein the benign gynecological neoplastic disease is occurrence of uterine fibroids.

15. An agent as specified in any one of the preceding claims.

Fig. 1

| Cell Type | Ectopic Peritoneal EM | Ectopic Peritoneal Adjacent EM | Ectopic Ovarian EM | Eutopic Endometrium (EM +ve) | Control (EM -ve) |
|---|---|---|---|---|---|
| Epithelial | 20 | 25 | 2 | 43 | 71 |
| Stromal | 80 | 75 | 98 | 57 | 29 |

Fig. 2

**Endometriosis Expressed Protein**

| Target | FAP |
|---|---|
| Example SEQ ID NO | 16 |

Linker
- -(G4S)x
- -(G2S)x
- -Ig Hinge sequence
- -Linker-CH2+CH3-linker
- -Linker-CH3-linker-Fc-linker

**Immune Cell recruiting moity or cytotoxic drug**

| Target | -CD3 -CD8 -CD16 -Cytotoxic drug |
|---|---|
| Example SEQ ID NO | 17 |

Linker
- -(G4S)x
- -(G2S)x
- -Ig Hinge sequence
- -No linker

Cleavable Linker
- -Sensitive to MMP 2, 9, 10, 26 cleavage
- -No linker

Linker
- -(G4S)x
- -(G2S)x
- -Ig Hinge seq
- -No linker

**Steric blocker and /or Half-life extending molecule**

| Target | Bulk serum protein e.g., Albumin Ferritin, Ig, FC, or an inert polypeptide |
|---|---|

Fig. 3

Antibody domain
targeting FAP

Antibody domain
targeting effector cells

Binding site Blocker (e.g.
serum binding domain
bidns to human serum in
blood)

Masking elements (Cleavable Linker and Binding
site Blocker) prevent binding to effector cells in the
wrong microenvironment

- Cleavable Linker for E126
- Cleavable Linker for E110
- Cleavable Linker for E107
- Cleavable Linker for E108

Fig. 4

**No debris**     **Single cells**     **Viable**

Fig. 4 (continued)

Fig. 4 (continued)

Fig. 5

PC568 FAP

PC568 Isotype

PC573 FAP

PC573 Isotype

PE564 FAP

PE564 Isotype

Qifi Calibration

beads

FITC-A

Fig. 5 (continued)

Fig. 5 (continued)

**FAP Antigen density**

Fig. 6

Fig. 6 (continued)

Fig. 6 (continued)

Fig. 7

| | MFI FAP |
|---|---|
| PE575 epithelial | 579 |
| PE576 epithelial | 509 |
| PE575 stromal | 26357 |
| PE576 stromal | 12108 |

| Stromal Cell population | % of FAP+ Cells |
|---|---|
| PE569 Ovarian Endometrioma | 67.3% |
| PE569 Deep infiltrating Endometriosis | 98.1% |
| PE571 Endometriosis/Peritoneal adhesions | 85.1% |

Fig. 8

Fig. 9

**Cytotoxicity, stromal:epithelial 1:1**

Legend:
- FMC2
- No BITE = T+E

x-axis: Treatment
y-axis: Cytotoxicity in %

\* No BITE control (target cells and effector cells only, cells continue to proliferate)

Fig. 10

Fig. 11

Fig. 12

Fig. 13

**Treatment**

● Target1scFV xCD3scFV

▲ FAPscFVxCD3scFV

■ Untreated T-cells and Target cells

Activation Marker: CD25+, CD107a, IFNg+

EP 4 260 907 A1

Fig. 14

**FAP+ myoma cells**

Fig. 15

MMP26 serum concentration

## EUROPEAN SEARCH REPORT

**Application Number**

EP 22 16 7625

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EL-ZAYADI AHMED A ET AL: "Anti-IL-6 receptor monoclonal antibody as a new treatment of endometriosis", IMMUNOLOGIC RESEARCH, vol. 68, no. 6, 17 September 2020 (2020-09-17), pages 389-397, XP037298267, ISSN: 0257-277X, DOI: 10.1007/S12026-020-09153-5 * figure 3; table 1 * | 1-9, 12-14 | INV. A61P15/00 C07K16/28 C07K16/40 |
| A | JANA SOSTOA ET AL: "Targeting the tumor stroma with an oncolytic adenovirus secreting a fibroblast activation protein-targeted bispecific T-cell engager", JOURNAL FOR IMMUNOTHERAPY OF CANCER, BIOMED CENTRAL LTD, LONDON, UK, vol. 7, no. 1, 25 January 2019 (2019-01-25), pages 1-15, XP021270677, DOI: 10.1186/S40425-019-0505-4 * figures 1a,2-6 * | 1-9, 12-14 | |
| A | WO 2020/118109 A2 (CYTOMX THERAPEUTICS INC [US]) 11 June 2020 (2020-06-11) * example 1; table 1 * | 1-9, 12-14 | |

TECHNICAL FIELDS SEARCHED (IPC)

A61P
C07K

-/--

The present search report has been drawn up for all claims

| Place of search | Date of completion of the search | Examiner |
|---|---|---|
| The Hague | 2 September 2022 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 22 16 7625

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anastasiu Costin Vlad ET AL: "Biomarkers for the noninvasive diagnosis of endometriosis: state of the art and future perspectives", International Journal of Molecular Sciences, 4 March 2020 (2020-03-04), XP055857589, DOI: 10.3390/ijms21051750 Retrieved from the Internet: URL:https://www.ncbi.nlm.nih.gov/pmc/articles/PMC7084761/pdf/ijms-21-01750.pdf [retrieved on 2021-11-03] * the whole document * | 1-9, 12-14 | |
| A | PENG YING ET AL: "Bioinformatics Analysis Identifies Molecular Markers Regulating Development and Progression of Endometriosis and Potential Therapeutic Drugs", FRONTIERS IN GENETICS, vol. 12, 4 August 2021 (2021-08-04), XP055956010, DOI: 10.3389/fgene.2021.622683 * the whole document * | 1-9, 12-14 | TECHNICAL FIELDS SEARCHED (IPC) |

The present search report has been drawn up for all claims

| Place of search | Date of completion of the search | Examiner |
|---|---|---|
| The Hague | 2 September 2022 | Cilensek, Zoran |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

EP 22 16 7625

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-9, 12(completely); 13, 14(partially)**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

Application Number

EP 22 16 7625

The Search Division considers that the present European patent application does not comply with the requirements of unity of invention and relates to several inventions or groups of inventions, namely:

```
1. claims: 1-9, 12(completely); 13, 14(partially)

     An agent comprising
     a. a ligand capable of specifically binding to FAP; and
     b. a therapeutic moiety, wherein the therapeutic moiety is
     capable of triggering cell death of cells expressing FAP on
     their cell surface;
     for use in treatment of a benign gynecological neoplastic
     disease.
     An agent comprising
     a. a ligand capable of specifically binding to FAP;
     b. a radioisotope label;
     for use in diagnosis of a benign gynecological neoplastic
     disease, wherein the radioisotope label is selected from the
     group comprising 94mTc, 186Re, 203Pb, 47Sc, 111In, 97Ru,
     62Cu, 88Y, 121Sn, 161Tb, 153Sm, 166Ho and 105Rh.
     Subject-matter related thereto.
                             ---


2. claims: 10, 11(completely); 13, 14(partially)

     An agent for use in treatment of a benign gynecological
     neoplastic disease, wherein the agent comprises
     a. a target protein binding moiety;
     b. an immune cell recruiting moiety;
     c. a masking moiety capable of shielding the immune cell
     recruiting moiety and/or the target protein binding moiety
     from binding to its target;
     d. a cleavable linker connecting the masking moiety with the
     other components of the agent, wherein the cleavable linker
     is specifically cleavable by matrix metalloproteinases
     (MMPs) 2, 9, 10, and/or 26, particularly wherein the
     cleavable linker is specifically cleavable by MMP2 or MMP9.
                             ---


3. claim: 15(partially)

     An agent as specified in any one of claims 1-9.
                             ---


4. claim: 15(partially)

     An agent as specified in any one of claims 10 and 11.
                             ---


5. claim: 15(partially)

     An agent as specified in claim 12.
                             ---
```

## ANNEX TO THE EUROPEAN SEARCH REPORT
## ON EUROPEAN PATENT APPLICATION NO.

EP 22 16 7625

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2022

| Patent document cited in search report | | Publication date | Patent family member(s) | | Publication date |
|---|---|---|---|---|---|
| WO 2020118109 | A2 | 11-06-2020 | AU 2019394972 | A1 | 03-06-2021 |
| | | | BR 112021010433 | A2 | 24-08-2021 |
| | | | CA 3120327 | A1 | 11-06-2020 |
| | | | CN 113271956 | A | 17-08-2021 |
| | | | EP 3890764 | A2 | 13-10-2021 |
| | | | IL 283756 | A | 29-07-2021 |
| | | | JP 2022512124 | A | 02-02-2022 |
| | | | KR 20210102318 | A | 19-08-2021 |
| | | | SG 11202105476X | A | 29-06-2021 |
| | | | TW 202039561 | A | 01-11-2020 |
| | | | US 2020377602 | A1 | 03-12-2020 |
| | | | WO 2020118109 | A2 | 11-06-2020 |

EPO FORM P0459

For more details about this annex : see Official Journal of the European Patent Office, No. 12/82

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 6750325 B1
- US 2012142611 A
- US 2016250341 A
- US 2016075767 A
- US 2015368302 A
- US 2011165621 A1

## Non-patent literature cited in the description

- **P VIGANO et al.** *Human Reproduction,* March 2018, vol. 33 (3), 347-352
- **SUN-WEI GUO ; PATRICK G GROOTHUIS.** *Human Reproduction Update,* September 2018, vol. 24 (5), 577-598
- **SAMBROOK et al.** Molecular Cloning: A Laboratory Manual. Cold Spring Harbor Laboratory Press, 2012
- **AUSUBEL et al.** Short Protocols in Molecular Biology. John Wiley & Sons, Inc, 2002
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Nat. Acad. Sci.,* 1988, vol. 85, 2444
- **ALTSCHUL et al.** *J. Mol. Biol.,* 1990, vol. 215, 403-410
- **STRYER.** Biochemistry. 21
- **SKERRA.** *Biochim. Biophys. Acta,* 2000, vol. 1482 (1-2), 337-50
- **KWAN et al.** *Structure,* 2003, vol. 11 (7), 803-813
- **VINCKE et al.** General strategy to humanize a camelid single-domain antibody and identification of a universal humanized nanobody scaffold. *J Biol Chem.,* 30 January 2009, vol. 284 (5), 3273-3284
- *Remington: the Science and Practice of Pharmacy,* ISBN 0857110624
- **L. LACHMAN et al.** The Theory and Practice of Industrial Pharmacy. 2013
- **SCHUBERTH et al.** *J Transl Med.,* 2013, vol. 11, 187
- **FANG J et al.** *Int J Cancer.,* 2016, vol. 138 (4), 1013-1023